(19)

Europäisches Patentamt
European Patent Office
Office européen des brevets

(11) **EP 4 625 423 A1**

(12) **EUROPEAN PATENT APPLICATION**

(43) Date of publication:
**01.10.2025 Bulletin 2025/40**

(21) Application number: **24315210.5**

(22) Date of filing: **23.04.2024**

(51) International Patent Classification (IPC):
***G16C 20/30*** (2019.01)      ***G16C 20/70*** (2019.01)

(52) Cooperative Patent Classification (CPC):
**G16C 20/30; G06N 5/01; G16H 20/10; G16H 50/20;**
**G16H 50/50; G16H 50/70; G16H 70/40;**
G16C 20/70

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **25.03.2024 EP 24290005**

(71) Applicant: **Sanofi**
**75017 Paris (FR)**

(72) Inventors:
• **MAVROUDIS, Panteleimon D**
**Cambridge, 02141 (US)**

• **PATIDAR, Krutika**
**Cambridge, 02141 (US)**
• **PILLAI, Nikhil S**
**Cambridge, 02141 (US)**
• **DHAKAL, Saroj**
**Cambridge, 02141 (US)**
• **AVERY, Lindsay**
**Cambridge, 02141 (US)**

(74) Representative: **Trichard, Louis**
**Venner Shipley LLP**
**200 Aldersgate**
**London EC1A 4HD (GB)**

(54) **METHOD, SYSTEM AND APPARATUS FOR CLASSIFYING MOLECULE DRUGS FOR DRUG OPTIMIZATION**

(57)     Methods, systems and apparatus are described for classifying one or more candidate molecule drugs in relation to one or more desired target conditions for use in downstream processing of drug optimization. A candidate molecule drug inference dataset is received for one or more molecule drugs, the candidate molecule drug dataset comprising data representative of a set of physiochemical values for each candidate molecule drug and a set of pharmacokinetic (PK) values associated with a dosage regimen and target in relation to each candidate molecule drug, and where each candidate molecule drug of the candidate molecule drug dataset is classified as a large molecule drug. For each candidate molecule drug of the candidate molecule drug inference dataset, the corresponding set of physiochemical values and set of PK values for the dosage regimen and target are applied to a machine learning (ML) model configured for classifying whether each candidate molecule drug meets one or more desired conditions associated with pharmacological or pharmacodynamic effects. The ML model is trained using an ML algorithm to train model parameters defining the ML model for classifying whether each candidate molecule drug meets the one or more desired conditions based on a training dataset comprising data representative of a plurality of molecule drugs, each molecule drug represented by a corresponding set of physiochemical values and a set of PK values associated with the dosage regimen and the target, and annotated in relation to corresponding pharmacological or pharmacodynamic effects, responses and/or values in relation to the desired conditions. A set of candidate molecule drugs satisfying the desired conditions is output for downstream processing of drug optimization.

FIG. 2A

EP 4 625 423 A1

## Description

### Field

This specification relates to methods, systems, and apparatus for classifying whether one or more molecule drugs meet one or more conditions associated with pharmacological / pharmacodynamic effects prior to downstream *in silico*, *in vitro* and/or *in vivo* analysis drug optimization, where each molecule drug is represented by: a set of physiochemical values, a set of pharmacokinetic (PK) values associated with a drug dosage regimen and target.

### Background

Monoclonal antibodies (mAb) as therapeutic molecule drugs have revolutionized healthcare to the point of becoming the best-selling drug format over the last few years. Recently, the pharmaceutical industry has shown a great interest in unveiling the pharmacokinetic (PK) principles of such antibodies since they provide crucial information regarding the efficacy of drugs and dosing regimens or strategies. During drug development, molecule drug candidates are often iteratively studied for key pharmacokinetic (PK) profiles and endpoints or parameters associated with absorption, distribution, metabolism, and excretion (ADME) or liberation ADME (LADME). Molecule drug (e.g., mAb) pharmacokinetics have been extensively studied with an additional focus on a drug's distribution and elimination properties. Among these, neonatal Fc receptor (FcRn) binding and molecular size have been shown to significantly affect the systemic PK of such antibodies. However, characteristics such as antibody charge and affinity to interact non-specifically with charged cell components are among a few properties that are yet to be fully understood. A quantitative relationship between the physicochemical properties of antibodies such as molecular weight, size, charge, FcRn binding affinity, and its PK response is still largely unknown but remains crucial for establishing an initial understanding of a drug's PK and desired pharmacological/pharmacodynamic effects such as, for example, target engagement or target occupancy.

During a molecule drug (e.g., mAb) discovery/optimization campaign, candidate molecule drugs with unacceptable PK characteristics in relation to a target are typically abandoned (or re-engineered). Due to budgetary restrictions and/or ethical concerns, neither *in vivo* testing, *in vitro* wet lab analysis, or high throughput screening can be conducted for the large swathe of possible candidate molecule drugs that meet certain desired physiochemical properties in relation to corresponding targets with certain desired physiochemical properties. There are numerous combinations of pairs of molecule drugs and targets and individually analyzing all possible pairs of molecule drug candidates and targets is impractical. Such brute force analysis would place extreme pressure on currently available computational and laboratory resources. This may cause significant delays and increase the costs for releasing promising new molecule drugs (e.g., mAb) and corresponding molecule drug treatments.

Thus, there is a desire for a molecule drug discovery mechanism or framework that minimizes or reduces not only the computational and laboratory resources required but also associated costs and delays for generating candidate molecule drugs (e.g., a short list of candidate molecule drugs) having the most promising PK characteristics and corresponding desired pharmacological/pharmacodynamic effects such as, for example, target engagement or occupancy values, efficacy and/or toxicity effects/endpoints prior to performing further downstream drug optimization processing such as *in vitro* and/or *in vivo* evaluation.

### Summary

According to a first aspect, there is provided a computer-implemented method of classifying one or more candidate molecule drugs in relation to one or more desired conditions associated with pharmacological or pharmacodynamic effects for drug optimization, the method comprising: receiving a candidate molecule drug inference dataset representing one or more candidate molecule drugs, wherein each candidate molecule drug in the candidate molecule drug dataset is represented by a set of physiochemical values for said candidate molecule drug and a set of pharmacokinetic (PK) values with a dosage regime and target in relation to said each candidate molecule drug and wherein each candidate molecule drug of the candidate molecule drug dataset is classified as a large molecule drug; applying, for each candidate molecule drug of the candidate molecule drug inference dataset, the corresponding set of physiochemical values and set of PK values for the dosage regimen and target to a machine learning (ML) model configured for classifying whether said each candidate molecule drug meets one or more desired conditions associated with pharmacological or pharmacodynamic effects, wherein the ML model is trained using an ML algorithm to train model parameters defining the ML model for classifying whether each candidate molecule drug meets the one or more desired conditions associated with pharmacological or pharmacodynamic effects based on a training dataset comprising data representative of a plurality of molecule drugs represented by known and/or computed sets of physiochemical values and sets of PK values associated with the dosage regimen and annotated based on the one or more desired conditions associated with pharmacological or pharmacodynamic effects; and outputting data representative of a set of candidate molecule drugs classified according to the desired conditions associated with pharmacological or pharmacodynamic effects for downstream processing for drug optimization.

The computer-implemented method of the first aspect, wherein each candidate molecule drug is an antibody. As an option, each candidate molecule drug is a monoclonal antibody. As another option, the monoclonal antibody has a size greater than or equal to 50KDa. As a further option, the monoclonal antibody is classified as a large molecule drug. As an option, the target is an antigen.

The computer-implemented method of the first aspect, wherein the set of physiochemical values for each molecule drug correspond with one or more physiochemical parameters or properties from the group of: molecular weight, MW; size or Stokes radius, ae; charge, Q; hydrophobicity; binding affinities; isoelectric point, pl; glycosylation; drug-target binding constant, KD; and any other type of physiochemical parameter or property associated with each molecule drug. As an option, the set of physiochemical properties for each molecule drug comprises at least MW, ae, Q, and KD.

The computer-implemented method of the first aspect, wherein the set of PK values of each molecule drug are associated with PK profiles, endpoints, parameters and/or properties corresponding with absorption, distribution, metabolism, and excretion, ADME, or liberation, absorption, distribution, metabolism, and excretion, LADME.

The computer-implemented method of the first aspect, wherein the PK profiles, endpoints, parameters and/or properties include one or more from the group of: dosage (D); dosing interval ($\tau$); maximum serum concentration ($C_{max}$); minimum time for $C_{max}$; minimum plasma concentration ($C_{min}$ or $C_{min,ss}$); average plasma concentration ($C_{av}$ or $C_{av,ss}$); concentration at steady state ($C_{ss}$); volume of distribution ($V_d$); absorption half-life; absorption rate constant; elimination half-life; time to reach minimum plasma concentration ($t_{min}$), total soluble ($T_{k,supp}$), membrane target suppressed ($Tm_{k,supp}$), elimination rate constant; area under the concentration time curve after a single dose, or in steady state ($AUC_{ss}$); Clearance (C); and any other type of PK profile, endpoint, parameter and/or property associated with ADME or LADME. As an option, the set of PK profiles, endpoints, parameters and/or properties for each molecule drug comprises at least $C_{min}$, $AUC_{ss}$, $C_{max}$, and $C_{ss}$. As an option, the set of PK profiles, endpoints, parameters and/or properties for each molecule drug comprises at least $C_{min}$, $AUC_{ss}$, $C_{max}$, $C_{ss}$, $T_{k,supp}$, and $Tm_{k,supp}$

The computer-implemented method of the first aspect, wherein the ML algorithm comprising one or more ML algorithms or combinations thereof from the group of: neural network based ML algorithm; decision tree-based classification ML algorithm; random forest decision tree ML algorithm; Adaptive Boosting based ML algorithm; Gradient Boosting based ML algorithm;

Bagging based ML algorithm; Ensemble based ML algorithm; any other supervised ML algorithm suitable for training model parameters of an ML model for outputting a classification whether one or more molecule drugs meet one or more desired conditions associated with pharmacological or pharmacodynamic effects when data representative of a set of physiochemical values and set of PK values for the target and dosage regime corresponding to the one or more molecule drugs are input.

The computer-implemented method of the first aspect, further comprising iteratively training model parameters defining the ML model using the ML algorithm associated with the ML model, the training comprising: receiving a training dataset associated with a plurality of molecule drugs, the training dataset comprising a plurality of training instances, each training instance associated with each molecule drug and comprising data representative of a known and/or desired set of physiochemical values and a corresponding known or computed set of PK values associated with the dosage regimen and the target, and annotated or labelled based on whether each molecule drug with target meets desired conditions associated with pharmacological or pharmacodynamic effects; iteratively applying the training dataset to the ML algorithm for training the model parameters of the ML model until a stopping criterion is reached; and outputting the model parameters representative of the trained ML model.

The computer-implemented method of the first aspect, wherein the sets of physiochemical values corresponding to the molecule drugs of the training dataset are based on synthetically generated molecule drugs generated over a desired range physiochemical values, wherein, for each synthetically generated molecule drug, the corresponding PK values associated with the dosage regimen and the target, and the associated with pharmacological or pharmacodynamic effects, responses and/or values corresponding to said each synthetically generated molecule drug are computed using a physiologically based pharmacokinetic, PBPK, model configured for simulating the effects of said synthetically generated molecule drug on the target in a cell system of a subject and outputting, for said each synthetically generated molecule, a corresponding set of PK values from selected PK endpoints and profiles and a corresponding pharmacological or pharmacodynamic effects, responses and/or values associated with the desired conditions.

The computer-implemented method of the first aspect, wherein the sets of physiochemical values, PK values associated with the dosage regimen and the target, and associated with pharmacological or pharmacodynamic effects, responses and/or values corresponding to each molecule drug of the training dataset are based on known or measured physiochemical values, PK values associated with the dosage regimen and the target, and associated with pharmacological or pharmacodynamic effects, responses and/or values of real-world molecule drugs.

The computer-implemented method of the first aspect, wherein the sets of physiochemical values corresponding to the molecule drugs of the training dataset are based on real-world molecule drugs meeting a desired range of physiochemical values, wherein, for each real-world molecule drug, the corresponding PK values associated with the target and dosage regimen and the pharmacological or pharmacodynamic effects, responses and/or values corresponding to said each real-world molecule drug are computed using a PBPK model configured for simulating the effects of said real-world molecule drug on the target in a physiological or cellular system of a subject and outputting, for said each real-world molecule drug, a corresponding set of PK values from selected PK endpoints and profiles and a corresponding pharmacological or pharmacodynamic effects, responses and/or values.

The computer-implemented method of the first aspect, wherein the sets of physiochemical' values corresponding to the molecule drugs of the candidate molecule drug inference dataset are based on synthetically generated molecule drugs generated over a desired range physiochemical values, wherein, for each synthetically generated molecule drug, the corresponding PK values associated with the dosage regimen and the corresponding pharmacological or pharmacodynamic effects, responses and/or values corresponding to said each synthetically generated molecule drug are computed using a PBPK model configured for simulating the effects of said synthetically generated molecule drug on the target in a physiological or cellular system of a subject and outputting, for said each synthetically generated molecule, a corresponding set of PK values from selected PK endpoints and profiles and corresponding pharmacological or pharmacodynamic effects, responses and/or values.

The computer-implemented method of the first aspect, wherein the sets of physiochemical values, PK values associated with the dosage regimen and the target, and the pharmacological or pharmacodynamic effects, responses and/or values corresponding to each molecule drug of the candidate molecule drug inference dataset are based on known or measured physiochemical values, PK values associated with the dosage regimen and the target, and pharmacological or pharmacodynamic effects, responses and/or values of real-world molecule drugs.

The computer-implemented method of the first aspect, wherein the sets of physiochemical values corresponding to the molecule drugs of the candidate molecule drug inference dataset are based on real-world molecule drugs meeting a desired range physiochemical values from a set of physiochemical parameters, wherein, for each real-world molecule drug, the corresponding PK values associated with the target and dosage regimen and the pharmacological or pharmacodynamic effects, responses and/or values corresponding to said each real-world molecule drug are computed using the PBPK model configured for simulating the effects of said real-world molecule drug on the target in a cell system of a subject and outputting, for said each real-world molecule drug, a corresponding set of PK values from selected PK endpoints and profiles and corresponding pharmacological or pharmacodynamic effects, responses and/or values.

The computer-implemented method of the first aspect, further comprising: receiving a set of physiochemical values corresponding to each molecule drug; receiving one or more sets of physiochemical values corresponding to the target; receiving a set of dosage regimens; generating, for each desired dosage regimen, one or more unique pairs of molecule drug and the target data based on pairing the set of desired physiochemical values of the molecule drug with each one of the one or more sets desired physiochemical values of the target; applying, for each dosage regimen, the set of desired physiochemical values of the molecule drug and the set of desired physiochemical values of the target for each pair of molecule drug and target data to the PBPK model; computing, for each dosage regimen and said each pair of molecule drug and target data, a set of PK values and the corresponding pharmacological or pharmacodynamic effects, responses and/or values associated with the desired conditions for said each pair of molecule drug and target data using the PBPK model; generating, for each pair of molecule drug and target and corresponding dosage regimen, either a training data instance comprising data representative of at least the set of physiochemical properties for each molecule drug, the computed set of PK values and annotated based on the corresponding pharmacological or pharmacodynamic effects, responses and/or values and the desired conditions computed for each pair of molecule drug and target and corresponding dosage regimen, or a candidate molecule drug inference data instance comprising data representative of at least the set of physiochemical properties for each molecule drug and the computed set of PK values computed for each pair of molecule drug and target and corresponding dosage regimen; storing the training data instances or candidate molecule drug inference data instances for use in training or inference/classification, respectively.

The computer-implemented method of the first aspect, wherein outputting the set of candidate molecule drugs satisfying the desired conditions associated with pharmacological or pharmacodynamic effects for downstream drug optimization further comprises outputting a shortlist of candidate molecule drugs, each candidate molecule drug represented by a corresponding set of physiochemical values and set of PK values for the dosage regimen and target for use in downstream processing including *in vitro* wet lab analysis or high-throughput screening.

The computer-implemented method of the first aspect, wherein outputting the set of candidate molecule drugs satisfying the desired conditions associated with pharmacological or pharmacodynamic effects for downstream drug optimization further comprises outputting a shortlist of candidate molecule drugs, each candidate molecule drug represented by a corresponding set of physiochemical values and a set of PK values for the dosage regimen and target for use in downstream processing including *in vivo* trials.

The computer-implemented method of the first aspect, wherein when one or more of the shortlist of candidate molecule drugs are represented by only a set of physiochemical values and a set of PK values, then prior to either *in vitro* wet lab analysis, or high-throughput screening, or *in vivo* trials, generating one or more real-world molecule drugs meeting the corresponding set of physiochemical values, set of PK values of the one or more candidate molecule drugs.

The computer-implemented method of the first aspect, wherein generating one or more real-world molecule drugs further comprising computing, for each corresponding candidate molecule drug, the structural and chemical properties required to realise or synthesise a real-world molecule drug meeting the corresponding set of physiochemical values, set of PK values and associated pharmacological or pharmacodynamic effects, responses and/or values.

The computer-implemented method of the first aspect, wherein outputting the set of candidate molecule drugs satisfying the desired conditions for downstream drug optimization further comprises outputting data representing either: a shortlist of candidate molecule drugs with PK values for the dosage regimen and target, and associated pharmacological or pharmacodynamic effects, responses and/or values meeting the desired conditions for review by one or more users or expert systems further assessing the output shortlist of candidate molecule drugs; or a list of candidate molecule drugs with PK values for the dosage regimen and target, and associated pharmacological or pharmacodynamic effects, responses and/or values classified as to whether or not they meet the desired conditions for review by one or more users or expert systems further assessing the output list of candidate molecule drugs.

According to a second aspect, there is provided an apparatus comprising a processor, a memory unit and a communication interface, wherein the processor is connected to the memory unit and the communication interface, wherein the processor and memory are configured to implement the computer-implemented method of the first aspect and any one or more features thereof.

According to a third aspect, there is provided a computer-readable medium comprising data or instruction code, which when executed on a processor, causes the processor to implement the computer-implemented method of the first aspect and any one or more features thereof.

According to a fourth aspect, there is provided a computing system comprising: a molecule and target generation unit configured for generating one or more molecules represented by corresponding sets of physiochemical values and one or more targets represented by desired physiochemical values; a physiologically based PK unit configured for generating a set of PK values and/or associated pharmacological or pharmacodynamic effects, responses, and/or values for each pair of molecule drug and targets as either training data instances or candidate molecule drug inference data instances; a ML module / unit configured for either training an ML model for classifying whether one or more molecule drugs meets desired conditions associated with pharmacological or pharmacodynamic effects for a particular dosage regimen using a training dataset of a plurality of training data instances, or inferring using a trained ML model to classify, for a particular dosage regimen, whether one or more input candidate molecule drug inference data instances meets the desired target conditions; and an output module / unit configured to output at least a shortlist of molecule drugs meeting the desired conditions associated with pharmacological or pharmacodynamic effects, each molecule drug represented by a set of physiochemical values and a set of PK values for use in generating real-world molecules with corresponding characteristics for *in vivo* trials, *in vitro* wet lab analysis, and/or high-throughput screening laboratory analysis.

The computing system of the fourth aspect, wherein the molecule and target generation unit, physiologically based PK unit, ML module / unit; and output module / unit are configured to implement the computer-implemented method of the first aspect and any one or more features thereof.

According to a fifth aspect, there is provided a non-transitory tangible computer-readable medium comprising data or instruction code for classifying one or more candidate molecule drugs in relation to one or more desired conditions associated with pharmacological or pharmacodynamic effects for drug optimization, which when executed on one or more processors, causes at least one of the processors to perform at least the operations of: receiving a candidate molecule drug inference dataset representing one or more candidate molecule drugs, wherein each candidate molecule drug in the candidate molecule drug dataset is represented by a set of physiochemical values for said candidate molecule drug and a set of PK values with a dosage regimen and target in relation to said each candidate molecule drug and where each candidate molecule drug of the candidate molecule drug dataset is classified as a large molecule drug; applying, for each candidate molecule drug of the candidate molecule drug inference dataset, the corresponding set of physiochemical values and set of PK values for the dosage regimen and target to a ML model configured for classifying whether said each candidate molecule drug meets one or more desired conditions associated with pharmacological or pharmacodynamic effects, wherein the ML model is trained using an ML algorithm to train model parameters defining the ML model for classifying whether each candidate molecule drug meets the one or more desired conditions based on a training dataset comprising data representative of a plurality of molecule drugs represented by known and/or computed sets of physiochemical values and sets of PK values associated with the dosage regimen and the target and annotated in relation to corresponding pharmacological or pharmacodynamic effects, responses and/or values in relation to the desired conditions; and outputting data representative of a set of candidate molecule drugs satisfying the desired conditions for downstream processing for drug optimization.

In various implementations, computer program instructions, optionally stored on a non-transitory computer readable medium which, when executed by one or more processors of a data processing apparatus, causes the data processing apparatus to carry out the program instructions to cause the one or more processors to perform operations comprising one or more aspects of the above- and/or below-described implementations (including one or more aspects of the appended claims).

In various implementations, apparatus are disclosed that comprise a computer readable storage medium having program instructions embodied therewith, and one or more processors configured to execute the program instructions to cause the apparatus to perform operations comprising one or more aspects of the above- and/or below-described implementations (including one or more aspects of the appended claims). The apparatus may comprise one or more processors or special-purpose computing hardware.

## Brief Description of the Drawings

So that the invention may be more easily understood, embodiments thereof will now be described by way of example only, with reference to the accompanying drawings in which: s

Figure 1a illustrates an example ML model classification process according to some embodiments of the invention;

Figure 1b illustrates an example ML model training process according to some embodiments of the invention;

Figure 1c illustrates an example candidate molecule shortlisting process according to some embodiments of the invention;

Figure 1d illustrates an example generation of a training dataset or candidate molecule drug inference dataset using a PBPK model according to some embodiments of the invention;

Figure 2a illustrates an example PBPK/ML system according to some embodiments of the invention;

Figure 2b illustrates an example decision tree of a trained tree-based ML model according to some embodiments of the invention;

Figure 2c illustrates a set of example result plots of the tree-based ML model classification of figures 2a and 2b according to some embodiments of the invention;

Figure 2d illustrates another set of example result plots for synthetic real-world molecule drugs for a tree-based ML model classification based on figures 2a to 2c according to some embodiments of the invention;

Figure 2e illustrates another set of example result plots for a selection of real-world molecule drugs for a tree-based ML model classification based on figures 2a to 2c according to some embodiments of the invention;

Figure 2f illustrates further sets of example result plots for multi-label classification when the tree-based ML model is trained for classifying three classes according to some embodiments of the invention;

Figure 2g illustrates another PBPK/ML system 250 according to some embodiments of the invention;

Figure 3 illustrates an example PBPK component 300 for implementing a mPBPK model 301 according to some embodiments of the invention;

Figure 4 is a schematic illustration of a system/apparatus for performing methods described herein.

Common reference numerals are used throughout the figures to indicate similar features.

## Detailed Description

Various example implementations described herein relate to method(s), apparatus, and system(s) for automatically, efficiently, and reliably performing *in silico* classification, predictions or estimates of whether candidate molecule drugs such as, without limitation, for example antibodies and/or monoclonal antibodies (mAb), meet various defined or desired conditions associated with pharmacological and/or pharmacodynamic effects (e.g., target occupancy conditions associated with target engagement response or target occupancy values) in which each candidate molecule drug (e.g., mAb) is represented by a set of physiochemical values, a set of PK values associated with a drug dosage regimen and a target (e.g., an antigen) in relation to the PK characteristics of said each molecule drug interacting with said target within a physiological or cellular system.

Pharmacological and/or pharmacodynamic effects include, without limitation, for example target engagement response and/or target occupancy and/or any other number of efficacy or toxicity-related endpoints (e.g., cytokine levels, biomarker concentration etc.). For example, the desired conditions may be based on, without limitation, for example target engagement or target occupancy conditions (e.g., percentage target occupancy (TO%) value $\leq$ upper TO% threshold and/or lower TO% threshold <= TO% value), where target occupancy may be considered as being the initial marker of pharmacological efficacy for a given molecule drug/target combination and can be independent of disease/clinical condition. Other desired conditions are also applicable and may be based on, without limitation, for example a number of efficacy or toxicity-related endpoints such as, without limitation, for example cytokine levels, biomarker concentration etc. and the like, and/or any other suitable pharmacological and/or pharmacodynamic effects. Each of target occupancy, efficacy or toxicity-related endpoints such as, without limitation, for example cytokine levels, biomarker concentration etc. may be used to form one or more desired conditions. An advantage of using target occupancy to form one or more desired conditions is because target occupancy is a marker that can be used for a variety of clinical conditions, whereas relevant efficacy-toxicity endpoints can be highly dependent on the disease of interest.

The set of physiochemical values for each molecule drug may correspond with one or more physiochemical parameters or properties from the group of: molecular weight (MW), size or Stokes radius (ae), charge (Q), hydrophobicity, FcRn binding affinity, isoelectric point (pl), glycosylation, drug-target binding constant (KD), and/or any other type of physiochemical parameter or property associated with each molecule drug. In some embodiments, the set of physiochemical values include, without limitation, for example at least the following properties of: MW, size or Stokes Radius (ae), Q, and KD. The set of PK values of each molecule drug are associated with PK profiles, endpoints, parameters and/or properties corresponding with ADME or LADME. The PK profiles, endpoints, parameters and/or properties include one or more from the group of: dosage (D) based on the desired dosage regimen, dosing interval ($\tau$) based on the desired dosage regimen, maximum serum concentration ($C_{max}$), minimum time for $C_{max}$, minimum plasma concentration ($C_{min,ss}$), average plasma concentration ($C_{av,ss}$), concentration at steady state ($C_{ss}$), volume of distribution or volume of distribution at steady state ($V_d$ or $V_{ss}$), absorption half-life, absorption rate constant, elimination half-life; half-life ($t_{1/2}$), time to reach minimum plasma concentration ($t_{min}$), total soluble ($T_{k,supp}$), membrane target suppressed ($Tm_{k,supp}$), elimination rate constant, area under the concentration-time curve after a single dose or in steady state ($AUC_{ss}$), Clearance (C) (unit mL/h/kg), and/or any other type of PK profile, endpoint, parameter and/or property associated with ADME or LADME as the application demands. In some example embodiments, the set of PK profiles, endpoints, parameters and/or properties for each molecule drug comprises at least $C_{min}$, $AUC_{ss}$, $C_{max}$, and $C_{ss}$. In some example embodiments, the set of PK profiles, endpoints, parameters and/or properties for each molecule drug comprises at least $C_{min}$, $AUC_{ss}$, $C_{max}$, $C_{ss}$, $t_{min}$, $T_{k,supp}$, $Tm_{k,supp}$.

The *in silico* classification, predictions or estimates are performed by a suitably trained ML model configured for classifying, predicting or estimating whether said candidate molecule drugs meet the various defined or desired conditions associated with pharmacological and/or pharmacodynamic effects (e.g., target engagement response or target occupancy). The ML model is trained using an ML algorithm and training dataset that includes a plurality of synthetic/virtual molecule drugs and/or real-world molecule drugs (e.g., mAb) in which each molecule drug in the training dataset is represented by at least data representative of the molecule drug including a corresponding set of physiochemical values and a set of PK values for each molecule drug associated with the drug dosage regimen and target interaction (e.g., an antigen) based on the PK characteristics of said each molecule drug and annotated according to how said each molecule drug satisfies the desired conditions associated with pharmacological and/or pharmacodynamic effects (e.g., target engagement response or target occupancy). The ML model is trained using a supervised training ML algorithm that computes model parameters defining the ML model in relation to the training dataset. For example, the ML algorithm may include one or more ML algorithms or combinations thereof from the group of: a neural network ML algorithm, a decision tree-based classification ML algorithm, a random forest decision tree ML algorithm, an Adaptive Boosting based ML algorithm, a Gradient Boosting based ML algorithm, a Bagging based ML algorithm, an Ensemble based ML algorithm, and/or any other supervised ML algorithm suitable for training model parameters of the ML model.

Once the model parameters defining the ML model are trained, the trained ML model is used for classifying a candidate molecule drug inference dataset that includes a plurality of synthetic/virtual molecule drugs and/or real-world molecule drugs (e.g., mAb) in which each molecule drug in the candidate molecule drug inference dataset is represented by at least data representative of a corresponding set of physiochemical values and a set of PK values for each molecule drug associated with the drug dosage regimen and target interaction (e.g., an antigen). The candidate molecule drug inference dataset is applied to and processed by the trained ML model for outputting data representative of classifying whether or not the candidate molecule drugs of the candidate molecule drug inference dataset satisfy the desired conditions associated with pharmacological and/or pharmacodynamic effects (e.g., target engagement response or target occupancy). For example, a shortlist of candidate molecule drugs may be obtained as the output based on those candidate molecule drugs of the candidate molecule drug inference dataset that satisfies or meets the desired conditions. The short list of candidate molecule drugs may be used in downstream drug optimization processes including, without limitation, for example *in vivo* testing, *in vitro* wet lab analysis or high throughput screening systems.

The training dataset and candidate molecule drug inference datasets may be generated by selecting data representative of molecule drugs having desired physiochemical characteristics, one or more targets of the same type having desired physiochemical characteristics and a dosage regimen, and computing and/or measuring the PK characteristics corresponding to the pairs of molecule drug and targets. For example, the data representative of each selected molecule drug includes a corresponding set of physiochemical values representing that molecule drug, where the set of physiochemical values for that molecule drug are within ranges of a set of physiochemical parameters set or selected by a user in relation to the molecule drug being evaluated. The data representative of each selected target includes a corresponding set of physiochemical values representing the target; where the set of physiochemical values for the target are within ranges of a set of physiochemical parameters set or selected by the user in relation to the targets being evaluated. This may result in a plurality of molecule drugs and a plurality of targets.

Once generated, a plurality of unique pairs of molecule drug and targets for the dosage regimen are derived from data representative of the selected molecule drugs and one or more targets. If the PK characteristics of a pair of molecule drug and target is unknown or not measured, then a physiologically based pharmacokinetic (PBPK) model configured for simulating the interaction of each molecule drug and target according to the dosage regimen in a modeled physiological system may be applied to output the PK characteristics of the interactions thereof. The PBPK model represents the molecule drug and target interactions by a plurality of mathematical relations that represent the cellular and tissue system of a physiological or cellular system and the interactions of each molecule drug and target according to the dosage regimen for the physiological or cellular system. The PBPK model computes or estimates a set of PK values for the molecule drug associated with the target and dosage regime. The set of PK values that is the output depends on the type of PBPK model and is based on a set of PK profiles and/or endpoints. The PBPK model may also calculate other pharmacological and/or pharmacodynamic effects, responses and values associated with the effect said each pair of molecule drug and target has on the physiological or cellular system. The pharmacological and/or pharmacodynamic effects, responses and values for each pair of molecule drug and target may be used to determine whether the corresponding molecule drug meets the desired conditions. The pharmacological and/or pharmacodynamic effects, responses and values for each pair of molecule drug and target may include, without limitation, for example corresponding target engagement response and target engagement/occupancy values, efficacy and/or toxicity related endpoints and the like. The pharmacological and/or pharmacodynamic effects, responses and values for each pair of molecule drug and target may be computed by the PBPK model and/or computed/estimated based on the PK values estimated from the PBPK model and the like.

As an option, a training dataset may be generated that includes a plurality of training data instances, each training data instance including data representative of a pair of molecule drug and target for each molecule drug of the plurality of molecule drugs and each target of the plurality of targets, in which each training data instance is annotated or labelled based on whether the pharmacological and/or pharmacodynamic effects, responses and values for each corresponding pair of molecule drug and target meets the desired conditions associated with pharmacological and/or pharmacodynamic effects. The training data instance representing the pair of molecule drug and target includes, for example, the set of physiochemical values representing the molecule drug, the set of PK values for the molecule drug associated with the target and drug dosage regimen, and an annotation or label associated with whether the corresponding one or more pharmacological and/or pharmacodynamic effects, responses and values for each pair of molecule drug and target (e.g., a target engagement or occupancy) meets the desired condition.

Additionally, a candidate molecule drug inference dataset may be generated that includes a plurality of candidate molecule drug data instances, each candidate molecule drug data instance including data representative of a pair of molecule drug and target for each molecule drug of the plurality of molecule drugs and each target of the plurality of targets. The candidate molecule drug data instance representing the pair of molecule drug and target includes, for example, the set of physiochemical values representing the molecule drug, the set of PK values for the molecule drug and associated with the target and drug dosage regimen.

Each of the training and/or candidate molecule drug inference datasets may be populated with data representative of real-world molecule drugs each having a known set of physiochemical values, and a known or measured set of PK values associated with the target and drug dosage regimen. The training dataset may be annotated based on whether the pair of molecule drug and target in each training data instance meets the desired conditions associated with pharmacological and/or pharmacodynamic effects. The required set of physiochemical values, and a known or measured set of PK values, and/or corresponding pharmacological and/or pharmacodynamic effects, responses and/or values for each molecule drug may be data mined from various databases and/or publications associated with evaluations of the real-world molecule drugs for generating either the training dataset or the candidate molecule drug inference dataset.

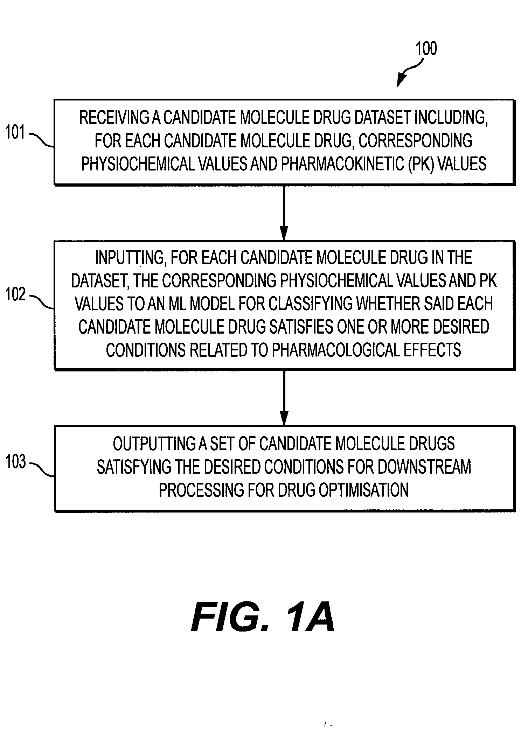

Figure 1a illustrates an example ML model classification process 100 classifying one or more candidate molecule drugs in relation to one or more desired target conditions for drug optimization. The ML model classification process 100 includes at least the following steps of:

In step 101, receiving a candidate molecule drug inference dataset representing one or more candidate molecule drugs. Each candidate molecule drug in the candidate molecule drug dataset is represented by a set of physiochemical values for said candidate molecule drug, a set of PK values associated with a dosage regimen and target in relation to said each candidate molecule drug, and a corresponding target engagement or occupancy value of said each candidate molecule drug.

In some examples, each candidate molecule drug is an antibody. In some embodiments, each candidate molecule drug is classified as a large molecule drug. In some embodiments, each candidate molecule drug is a monoclonal antibody (mAb). In some embodiments, each candidate molecule drug is an mAb that is classified as a large molecule drug. In an example, a large mAb may be a regular sized full-length mAb with a MW in the region of, without limitation, for example 50 kDa to 150 kDa. In another example, a large molecule drug or large mAb may be considered to be a molecule drug or mAb with a size greater than or equal to 50 kDa. A candidate molecule drug may also be classified as a large molecule drug based on therapeutic treatment or desired effect in which smaller-sized antibody/antibody fragments having an MW in the region of, without limitation, for example 3 kDa to 100 kDa, or 50 kDa to 150 kDa. In some embodiments, the candidate molecule drug corresponds to any sized mAb or mAb fragment that a corresponding PBPK model is capable of using to generate/estimate PK values and/or pharmacological or pharmacodynamic effects, responses and/or values (e.g., target occupancy values) and the like. The target is, without limitation, for example an antigen or any other molecule that a drug is designed to bind to.

In step 102, applying, for each candidate molecule drug of the candidate molecule drug inference dataset, data representative of the corresponding set of physiochemical values and set of PK values for the dosage regimen and target a machine learning (ML) model configured for classifying whether said each candidate molecule drug meets one or more desired conditions associated with pharmacological and/or pharmacodynamic effects (e.g., target occupancy conditions, efficacy-toxicity conditions, and the like). For example, the ML model is trained using an ML algorithm to train model parameters defining the ML model for classifying whether each candidate molecule drug meets the one or more desired conditions associated with pharmacological and/or pharmacodynamic effects based on a training dataset including data representative of a plurality of molecule drugs represented by known and/or computed sets of physiochemical values and sets of PK values associated with the dosage regimen and the target, and annotated with corresponding pharmacological and/or pharmacodynamic effects, responses or values in relation to the desired conditions.

In some embodiments, the ML algorithm may include one or more ML algorithms or combinations thereof from the group of: a neural network ML algorithm, a decision tree-based classification ML algorithm, a random forest decision tree ML algorithm, an Adaptive Boosting based ML algorithm, a Gradient Boosting based ML algorithm, a Bagging based ML algorithm, an Ensemble based ML algorithm, and/or any other supervised ML algorithm suitable for training model parameters of the ML model accordingly.

In step 103, outputting data representative of a set of candidate molecule drugs classified according to the desired conditions associated with pharmacological and/or pharmacodynamic effects for downstream processing for drug optimization. For example, a shortlist of candidate molecule drugs may be output from ML model that includes data representative of a set of candidate molecule drugs that satisfy or meet the desired conditions associated with pharmacological and/or pharmacodynamic effects for downstream processing for drug optimization.

In some embodiments, outputting the set of candidate molecule drugs satisfying the desired conditions associated with pharmacological and/or pharmacodynamic effects for downstream drug optimization may include outputting a shortlist of candidate molecule drugs, each candidate molecule drug represented by a corresponding set of physiochemical values and set of PK values for the dosage regimen and target for use in downstream processing including *in vitro* wet lab analysis or high-throughput screening. Alternatively or additionally, in some embodiments, outputting the set of candidate molecule drugs satisfying the desired conditions associated with pharmacological and/or pharmacodynamic effects for downstream drug optimization further includes outputting a shortlist of candidate molecule drugs, each candidate molecule drug represented by a corresponding set of physiochemical values and a set of PK values for the dosage regimen and target for use in downstream processing including *in vivo* trials.

Should the classified one or more of candidate molecule drugs or one or more of the short list of candidate molecule drugs be represented by only data representative of a set of physiochemical values and a set of PK values, and/or associated with pharmacological and/or pharmacodynamic effects, responses, values (e.g., target engagement or occupancy values), then prior to either *in vitro* wet lab analysis, or high-throughput screening, or *in vivo* trials, one or more real-world molecule drugs may be generated, computed and/or found that meet the corresponding set of physiochemical values and set of PK values and the desired conditions associated pharmacological and/or pharmacodynamic effects (e.g., target engagement or occupancy values meeting the target occupancy conditions) of the one or more candidate molecule drugs. For example, one or more real-world molecule drugs may be generated based on computing, for each corresponding candidate molecule drug, the structural and chemical properties required to realise or synthesize a real-world molecule drug meeting the corresponding set of physiochemical values, set of PK values and associated pharmacological and/or pharmacodynamic effects (e.g., target engagement or occupancy value). Thus, a shortlist of candidate real-world molecule drugs may be sent to downstream drug optimization processes such as, for example, *in vitro* wet lab analysis, high throughput screening and/or *in vivo* trials and the like.

The ML model for classifying whether a candidate molecule drug meets one or more desired conditions associated with pharmacological and/or pharmacodynamic effects may be trained based on an ML algorithm using supervised learning. Examples of ML algorithms or processes that may be used to train model parameters defining the ML model include or may be based on, without limitation, for example, any ML algorithm or process that can train model parameters on a labelled training datasets of molecule drugs represented by a set of physiochemical values and a set of PK values associated with the dosage regimen and target, and annotated / labelled using the corresponding pharmacological and/or pharmacodynamic responses/values in relation to the one or more desired conditions associated with the corresponding pharmacological and/or pharmacodynamic effects (e.g., labels are assigned to each molecule drug based on the corresponding target engagement or occupancy value meeting one or more desired target occupancy conditions), which may be used to classify whether one or more molecule drugs meet the one or more desired conditions.

Some examples of ML algorithms may include or be based on, by way of example only but is not limited to, one or more ML algorithms associated with random forests, decision tree learning, association rule learning, Adaptive Boosting (AdaBoost) based ML algorithms, Gradient boosting based ML algorithms, extreme Gradient boosting (XGBoost) based ML algorithms, Bagging based ML algorithms, bootstrap aggregating, CoBoost, BrownBoost, data mining algorithms/-methods, artificial neural networks (NNs), feed forward NNs, recursive NNs, deep NNs, deep learning, deep learning ANNs, convolutional NNs, support vector machines (SVMs), one or more combinations thereof or modifications thereto and the like and/or any other suitable ML algorithm as the application demands. The selection of the hyperparameters for the ML algorithm that affects training the resulting ML model may be based on performing a hyperparameter grid search over a multiple hyperparameter ranges.

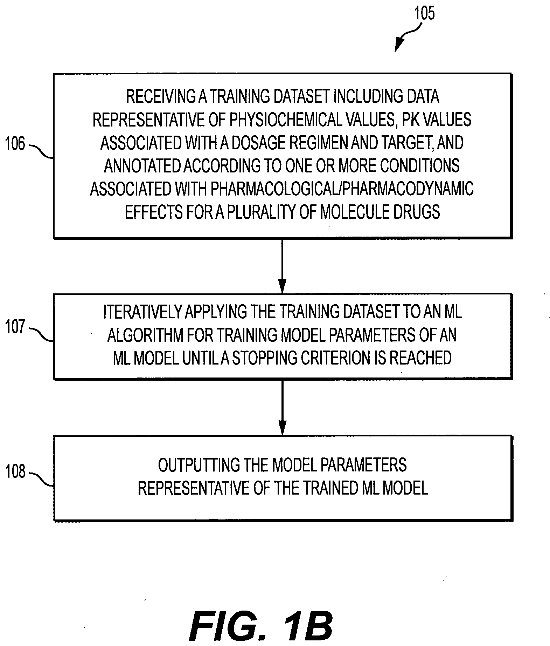

Figure 1b illustrates an example ML model training process 105 for using an ML algorithm for training model parameters defining the ML model for classifying whether one or more molecule drugs meet or satisfy one or more desired conditions associated with pharmacological or pharmacodynamic effects (e.g., one or more target occupancy conditions). It is assumed that the ML algorithm is selected for use in classifying the one or more molecule drugs. The training dataset includes a plurality of training data instances, each training data instance corresponding to a molecule drug and target pair of the plurality of molecule drugs and targets that may be generated based on desired ranges of a desired set of physiochemical parameters for the molecule drugs and targets, where each molecule drug and target pair is annotated using the corresponding pharmacological and/or pharmacodynamic response/values and the one or more desired conditions. It is assumed that the training dataset corresponds to a particular dosage regimen. Thus, if multiple dosage regimens are to be evaluated, a corresponding multiple of ML models will need to be trained (e.g., an ensemble of ML models) for each dosage regimen. The trained ML model defined by the output model parameters is then used by the ML model process 100 of figure 1a. The ML model training process 105 includes iteratively training model parameters defining the ML model using the ML algorithm associated with the ML model based on the following steps of:

In step 106, receiving a labelled training dataset associated with a plurality of molecule drugs, the training dataset including a plurality of training instances, each training instance associated with each molecule drug and including data representative of a known and/or desired set of physiochemical values representing said each molecule drug and a corresponding known or computed set of PK values associated with the dosage regimen and the target said each molecule drug interacts with, and which are annotated or labelled according to one or more desired conditions associated with pharmacological and/or pharmacodynamic effects. For example, each training instance is annotated by comparing the corresponding pharmacological and/or pharmacodynamic responses/values for said each molecule drug / target pair with the one or more desired conditions associated with said pharmacological and/or pharmacodynamic effects. For example, a first label may be assigned to a molecule drug when a target engagement or occupancy value corresponding to the molecule drug meets or satisfies a desired target occupancy condition, or a second label is assigned to the molecule drug when the target engagement or occupancy value corresponding to the molecule drug does not meet the desired target occupancy condition. The annotation and/or labelling may include two or more labels corresponding to two or more classification classes based on the number of target occupancy conditions that are required and/or as the application demands.

In some examples, each training data instance of a molecule drug may be represented as a row of one or more lists or tables (e.g., a comma-separated file format (csv)). if multiple tables/lists are used, each row may be linked to form each training data instance of the molecule drug. For example, the physiochemical values or drug-target candidate properties of the molecule drug for said each training data instance may be stored in a first list or table in separate columns such as, without limitation, for example target baseline, target half-life, and drug binding affinity (KD). This may be in any order. As well, for each training data instance of a molecule drug, the corresponding set of PK values and one or more pharmacological/pharmacodynamic values (e.g., target occupancy % value) associated with the molecule drug of the training data instance may be stored in a row of another second list or table (e.g., stored in another csv file) with separate columns representing the different PK values and the one or more pharmacological/pharmacodynamic values (e.g., target occupancy % value) of the set of PK values and one or more pharmacological/pharmacodynamic values (e.g., target occupancy % value). Each row in this second list or table may be include a further column for annotating based on whether the corresponding one or more pharmacological/pharmacodynamic values (e.g., target occupancy value) meet the desired conditions associated with the corresponding pharmacological/pharmacodynamic effects. For example, the set of PK values associated with the molecule drug is stored as a row in the second list / table in which each PK value of a PK property is stored in a separate column of the row, in which multiple columns include, without limitation, for example AUC at steady state, Cmin of drug, soluble target suppression, membrane target suppression, minimum target occupancy in tight, leaky, and plasma, mean target occupancy in tight, leaky, and plasma, maximum target occupancy at steady state in tight, leaky, and plasma, minimum target occupancy at steady state in tight, leaky, and plasma. Each of the pharmacological/pharmacodynamic values may also be stored in separate columns, along with corresponding columns containing annotations associated with whether the pharmacological/pharmacodynamic values meet the desired conditions. The corresponding rows of the first and second lists / tables may be used or linked to form a training data instance of the molecule drug.

In some embodiments, each training data instance for each molecule drug may be preprocessed and represented as, without limitation, for example a vector or embedding of the data representative of the known and/or desired set of physiochemical values representing said each molecule drug, the corresponding known or computed set of PK values associated with the dosage regimen and the target said each molecule drug interacts with, and the annotations or labels associated with the corresponding pharmacological/pharmacodynamic values (e.g., target engagement or occupancy value) corresponding to said each molecule drug.

In step 107, iteratively applying the labelled training dataset to the ML algorithm for training the model parameters of the ML model until a stopping criterion is reached. For example, the stopping criterion may be based on one or more from the group of: a maximum number of iterations or passes of the training dataset is reached; an error threshold associated with the output classifications is reached.

When the ML algorithm is based on decision trees or a tree-based ML algorithm, the ML model represents a decision tree comprising is a non-cyclic tree structure including a root node, a plurality of decision nodes, and terminal nodes connected via labelled edges or branches, where the root node includes a first rule or decision that splits into one or more of a decision node and/or a terminal node, and each decision node including a further rule or decision that splits via labelled branches corresponding to the decision into one or more of another decision node or terminal node. Each terminal node outputting a classification accordingly. The stopping criterion may include, without limitation, for example, predetermined constraint on the tree size including, for example, providing a minimum number of samples for a node split, deploying the minimum number of samples for a terminal node, allowing maximum depth of tree (e.g., vertical depth), maximum number of terminal nodes, maximum number of features to consider for the split, impurity criterion.

In step 108, outputting the model parameters representative of the trained ML model.

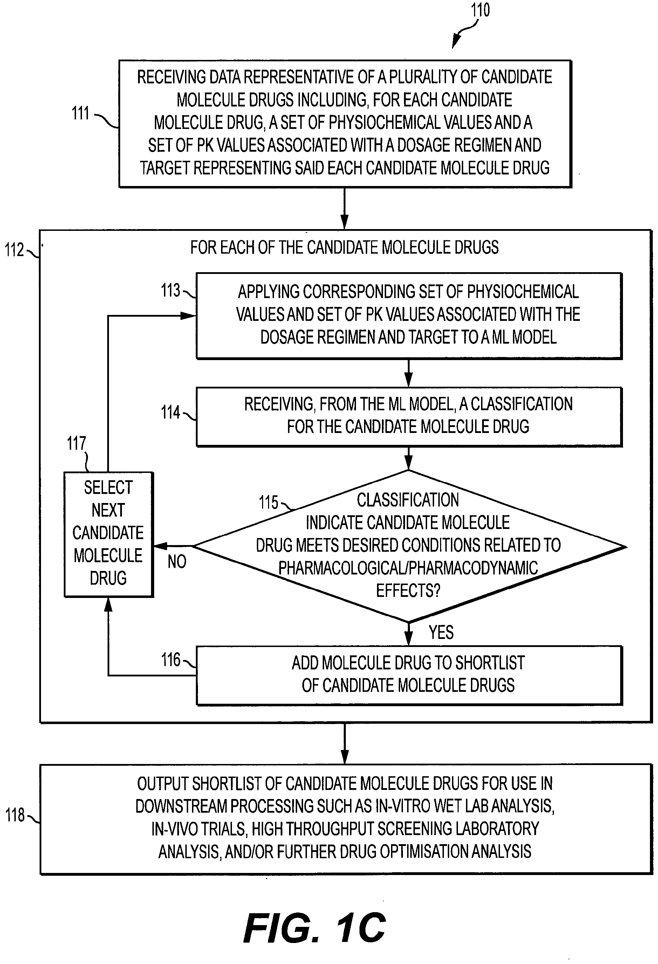

Figure 1c illustrates an example molecule drug shortlisting process 110 using the trained ML model process 100 for classifying a candidate molecule drug inference dataset including a plurality of molecule drugs that meet one or more desired conditions associated with pharmacological/pharmacodynamic effects. It is assumed that the ML model has been generated in accordance with the ML model training process 105 of figure 1b. The molecule drug shortlisting process 110 includes the following steps of:

In step 111, receiving a candidate molecule drug inference dataset including data representative of a plurality of candidate molecule drugs. Each of the candidate molecule drugs represented by a corresponding set of physiochemical values and a corresponding set of PK values associated with a dosage regimen and target.

In step 112, processing each molecule drug of the candidate molecule drug inference dataset to determine whether said each molecule drug meets the one or more desired conditions (e.g., one or more target occupancy conditions) for inclusion into the shortlist. Each candidate molecule drug from the candidate molecule drug inference dataset is selected for testing/evaluation based on, for each candidate molecule drug, the following steps of:

In step 113, applying the corresponding set of physiochemical values and set of PK values associated with the dosage regimen and target to the trained ML model (e.g., ML model from ML model process 110 or the ML model output by process 105 of figures 1a or 1b) configured for classifying whether said a molecule drug meets the one or more desired conditions (e.g., target occupancy conditions) specified when training said ML model.

In step 114, receiving, from the ML model, data representative of a classification of whether the molecule drug meets the one or more desired conditions (e.g., one or more target occupancy conditions).

In step 115, determining whether the classification indicates the candidate molecule drug meets the one or more desired conditions associated with pharmacological/pharmacodynamic effects (e.g., target occupancy condition(s)) required for shortlisting said each candidate molecule drug. If the classification indicates the molecule drug meets the one or more desired conditions (e.g., target occupancy conditions) (e.g., Y), then proceed to step 116, otherwise (e.g., N) proceed to step 117 for selecting the next molecule drug from the candidate molecule drug inference dataset. If there are no further molecule drugs in the candidate molecule drug inference dataset, then proceed to step 118 or, if no molecule drugs meet the target conditions, then terminate the shortlisting process 110.

In step 116, adding the molecule drug to a shortlist of candidate molecule drugs. Proceed to step 117.

In step 117, selecting the next candidate molecule drug from the candidate molecule drug inference dataset and the process 110 proceeds to step 113 for testing the selected candidate molecule drug.

In step 118, outputting the shortlist of candidate molecule drugs for use in downstream processing such as *in vitro* wet lab analysis including, for example, high throughput screening laboratory analysis for analysis of the efficacy and/or toxicity and the like, *in vivo* trials, and/or further drug optimization analysis or processes.

As previously described, the training dataset and candidate molecule drug inference datasets may be generated by selecting data representative of molecule drugs having desired physiochemical characteristics, one or more targets of the same type having desired physiochemical characteristics and a dosage regimen, however, the PK characteristics for the pairs of molecule drugs and targets may not be known, measured or available. Thus, the PK characteristics may be computed by simulating the interaction between each pair of molecule drug and target in a physiological or cellular system. For example, a PBPK model may be applied for estimating or computing the PK characteristics and/or one or more corresponding pharmacological/pharmacodynamic values for use with the one or more desired conditions for each pair of molecule drug and target for a particular dosage regimen.

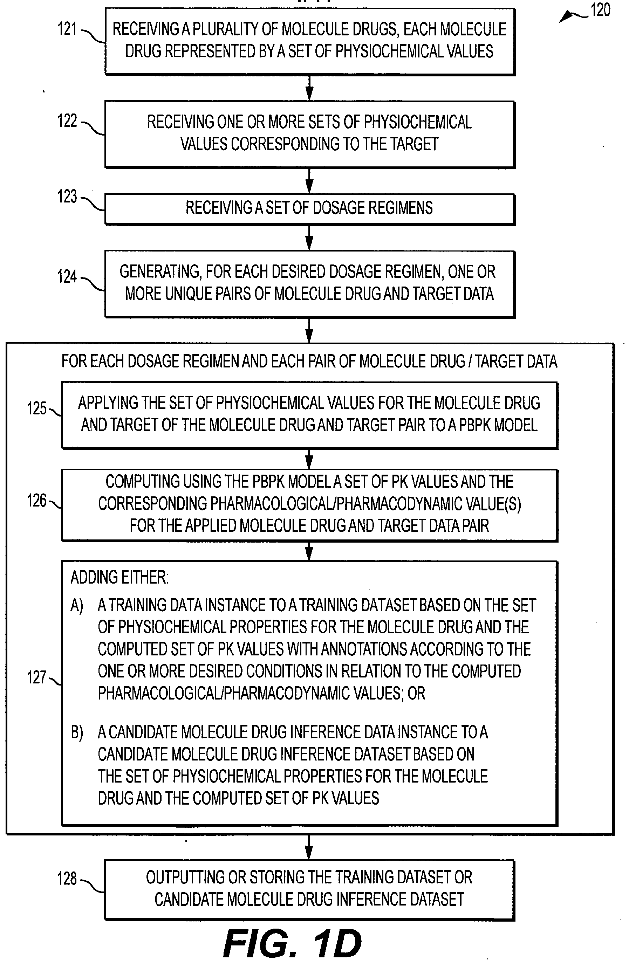

Figure 1d describes a PBPK process 120 for computing the PK characteristics required to generate the training or candidate molecule drug inference datasets for use by processes 100, 105 and 110 of figures 1a to 1c and/or as described herein. The PBPK process 120 includes the following steps of:

In step 121, receiving a plurality of molecule drugs, each molecule drug represented by a set of physiochemical values. For example, a user may specify that the molecule drugs for evaluation should meet a set of physiochemical values within desired ranges of a set of physiochemical characteristics or properties, where a plurality of molecule drugs are generated each represented by a unique set of physiochemical values within the desired range for each physiochemical characteristic for each molecule drug in the desired set of physiochemical characteristics/properties.

In step 122, receiving one or more sets of physiochemical values corresponding to the target. For example, a user may specify that the targets for evaluation should meet a set of physiochemical values within desired ranges of a set of physiochemical characteristics or properties, where a plurality of targets are generated each represented by a unique set of physiochemical values within the desired range for each physiochemical characteristic for each target in the desired set of physiochemical characteristics/properties.

in step 123, receiving a set of dosage regimens. These may be specified by a user in the appropriate dosage regimen format.

In step 124, generating, for each desired dosage regimen, one or more unique pairs of molecule drug and the target data based on pairing the set of desired physiochemical values of each molecule drug with each of the one or more sets desired physiochemical values of the target;

In step 125, applying, for each dosage regimen, the set of desired physiochemical values of the molecule drug and the set of desired physiochemical values of the target for each pair of molecule drug and target data to a PBPK model.

In step 126, computing, for each dosage regimen and said each pair of molecule drug and target data, using the PBPK model a set of PK values and the corresponding pharmacological/pharmacodynamic values (e.g., target engagement or occupancy value) associated for said each pair of molecule drug and target data.

In step 127, adding, for each pair of molecule drug and target and corresponding dosage regime, either: a) a training data instance for a training dataset; or b) a candidate molecule drug inference data instance for a candidate molecule drug inference dataset; each training data instance including data representative of at least the set of physiochemical properties for each molecule drug, the computed set of PK values and pharmacological/pharmacodynamic values computed for each pair of molecule drug and annotated according to the one or more desired conditions in relation to the computed pharmacological/pharmacodynamic values (e.g., target engagement or occupancy value). Each candidate molecule drug data instance including data representative of at least the set of physiochemical properties for each molecule drug and the computed set of PK values computed for each pair of molecule drug and target, and corresponding dosage regimen.

In step 128, storing the training data instances in a training dataset or candidate molecule drug inference data instances in a candidate molecule drug inference dataset for use in training or inference/classification, respectively.

For example, the data representative of each selected molecule drug includes a corresponding set of physiochemical values representing that molecule drug, where the set of physiochemical values for that molecule drug are within ranges of a set of physiochemical parameters set or selected by a user in relation to the molecule drug being evaluated. The data representative of each selected target includes a corresponding set of physiochemical values representing the target, where the set of physiochemical values for the target are within ranges of a set of physiochemical parameters set or selected by the user in relation to the targets being evaluated. This may result in a plurality of molecule drugs and a plurality of targets.

The training dataset and candidate molecule drug inference datasets may be generated by selecting data representative of molecule drugs having desired physiochemical characteristics, one or more targets of the same type having desired physiochemical characteristics and a dosage regimen, and computing and/or measuring the PK characteristics of the corresponding pairs of molecule drug and target interactions. For example, the data representative of each selected molecule drug includes a corresponding set of physiochemical values representing that molecule drug, where the set of physiochemical values for that molecule drug are within ranges of a set of physiochemical parameters set or selected by a user in relation to the molecule drug being evaluated. The data representative of each selected target includes a corresponding set of physiochemical values representing the target, where the set of physiochemical values for the target are within ranges of a set of physiochemical parameters set or selected by the user in relation to the targets being evaluated. This may result in a plurality of molecule drugs and a plurality of targets.

Once generated, a plurality of unique pairs of molecule drug and targets for the dosage regimen are derived from data representative of the selected molecule drugs and the one or more targets. If the PK characteristics of a pair of molecule drug and target is unknown or not measured, then a physiologically based pharmacokinetic (PBPK) model configured for simulating the interaction of each molecule drug and target according to the dosage regimen in a modelled physiological system may be applied to output the PK characteristics of the interactions thereof. The PBPK model represents the molecule drug and target interactions by a plurality of mathematical relations that represent the cellular and tissue system of the physiological or cellular system and the interactions of each molecule drug and target according to the dosage regimen for the physiological or cellular system. The PBPK model computes or estimates a set of PK values for the molecule drug associated with the target and dosage regimen. The set of PK values that is output depends on the type of PBPK model and are based on a set of PK profiles and/or endpoints. Further the PBPK model may also be configured to compute or estimate corresponding pharmacological/pharmacodynamic values (e.g., target engagement/occupancy values) as defined by and computed by the PBPK model. The pharmacological/pharmacodynamic values may be used to annotate each molecule drug and target pair of the training dataset in respect of the one or more desired conditions associated with corresponding pharmacological/pharmacodynamic effects.

As an option, the training dataset may be generated that includes a plurality of training data instances, each training data instance including data representative of a pair of molecule drug and target for each molecule drug of the plurality of molecule drugs and each target of the plurality of targets and annotated according to the corresponding computed pharmacological/pharmacodynamic values and the desired conditions. The training data instance representing the pair of molecule drug and target includes, for example, the set of physiochemical values representing the molecule drug and the set of PK values for the molecule drug and associated with the target and drug dosage regimen, and the corresponding annotations in respect of the pharmacological/pharmacodynamic values (e.g., target engagement or occupancy values) in relation to the one or more desired conditions associated with corresponding pharmacological/-pharmacodynamic effects. Alternatively, as another option, the candidate molecule drug inference dataset may be generated that includes a plurality of candidate molecule drug data instances, each-candidate molecule drug data instance including data representative of a pair of molecule drug and target for each molecule drug of the plurality of molecule drugs and each target of the plurality of targets. Each candidate molecule drug data instance includes, for example, the set of physiochemical values representing the candidate molecule drug and the set of PK values for the candidate molecule drug and associated with the target and drug dosage regimen.

Each of the processes 100, 105, 110, 120 of figures 1a-1d may be implemented on an apparatus including a processor, a memory unit, and a communication interface. The processor is connected to the memory unit and the communication interface. The processor and memory may be configured to implement each of the processes 100, 105, 110, 120 and/or other processes described herein as computer-implemented methods. The processes 100, 105, 110, 120 and/or combinations thereof, modifications thereto based on one or more other processes as herein described may be stored on a computer-readable medium or non-transitory computer-readable medium. The computer-readable medium may include data or instruction code, which when executed on a processor, causes the processor to implement one or more of the processes 100, 105, 110, 120 and/or combinations thereof, modifications thereto as one or more computer-implemented methods as described herein.

In some embodiments, the molecule drugs of the training dataset and/or candidate molecule drug inference dataset are monoclonal antibodies (mAb) and/or mAb fragments. However, any type of antibody may be used: It may be preferred that the mAb are large molecule drugs.

Although the following description describes an PBPK and ML modelling system based on the processes 100, 105, 110 and 120 of figures 1a to 1d and application of the ML model thereof in relation to large molecule drugs (e.g., mAbs) and corresponding antigens (e.g., Ag), this is by way of example only and the invention is not so limited, it is to be appreciated by the skilled person that the principles described herein in relation to using a PBPK model and generating an ML model may be applied to any type of molecule drug and target pair as the application demands.

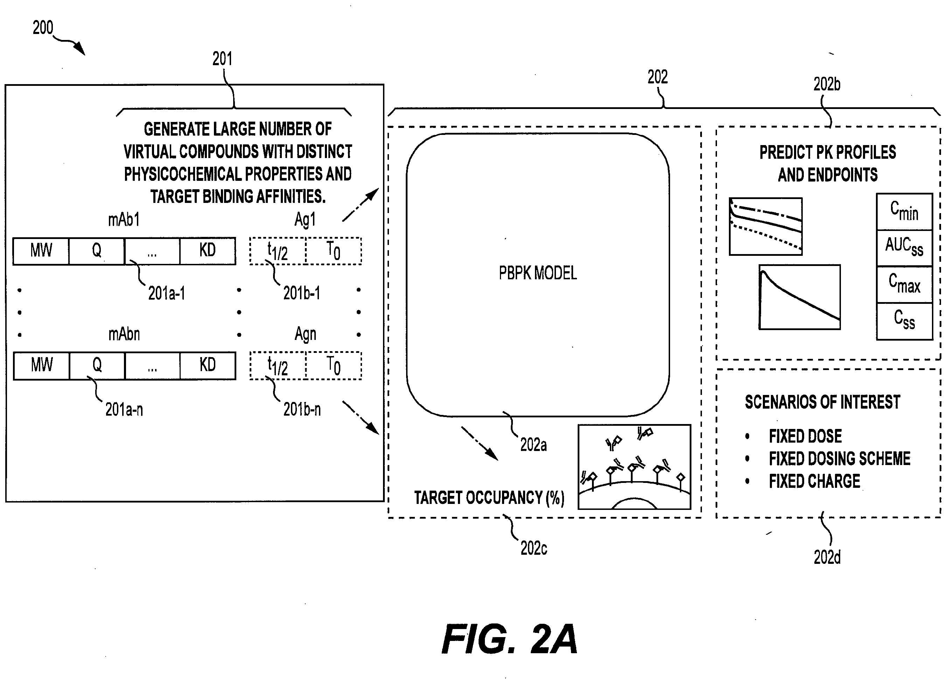

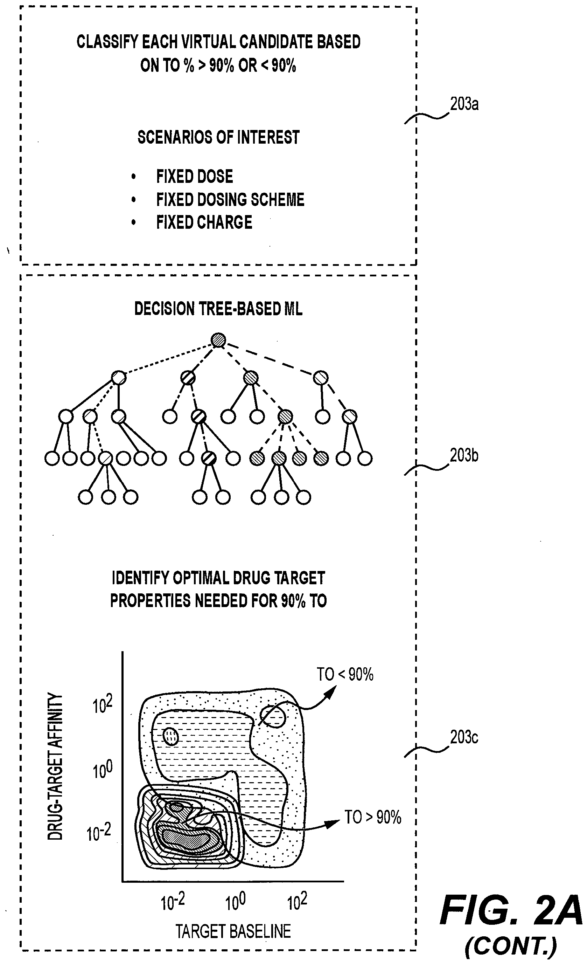

Figure 2a is an example PBPK and ML model system 200 for use in generating training and/or candidate molecule drug inference datasets using the PBPK model, for training the ML model using the training dataset to classify whether candidate molecule drugs satisfy desired conditions associated with pharmacological and/or pharmacodynamic effects, and for using the trained ML model to classify the candidate molecule drug inference dataset accordingly. In this example, the pharmacological and/or pharmacodynamic effects used is the target engagement or target occupancy and the one or more desired conditions are one or more desired target occupancy conditions. In this example, the candidate molecule drugs are monoclonal antibodies (mAb), the targets are antigens (Ag), and the desired target occupancy conditions are to classify whether each candidate molecule drug has a target occupancy value (TO%) greater than or less than 90% (e.g., $TO\% \geq 90\%$). Although the desired target occupancy condition is described as being TO% s 90%, this is by way of example only and the desired target occupancy conditions is not so limited, the skilled person in the art would appreciate that any type of target occupancy condition may be defined such as, for example, TO value greater than or equal to a first TO threshold value, TO value less than or equal to a second TO threshold value, and/or any one or more rules associated with TO values/thresholds suitable for shortlisting candidate drugs for downstream drug optimization, and the like. In this example, each candidate mAb is represented by the following set of physiochemical properties including, without limitation, for example at least an MW, an antibody charge (Q), and a binding constant value (KD) and each target Ag is represented by the following set of physiochemical properties including, without limitation, for example at least a target half-life ($t_{1/2}$) and target baseline ($T_0$). The PBPK and ML model system 200 combines a PBPK model 202a and ML model 203b to infer the optimal drug and target characteristics responsible for desired target engagement and occupancy. This can be used to determine the relationship between physicochemical properties of the large molecule drugs (e.g., mAbs) like charge and drug-target binding ($K_D$), ADME characteristics, and target properties like target baseline ($T_0$), half-life ($t_{1/2}$), and target receptor form.

The PBPK and ML system 200 includes a molecule generation unit 201, a PBPK model unit 202, and an ML classification unit 203. The PBPK and ML system 200 is configured for model-based assessment of target engagement response of antibody candidates (candidate molecule drugs) by integrating a PBPK model 202a with a tree-based ML model 203a. In the PBPK and ML system 200, the molecule generation unit 201 generates a plurality of molecule drugs represented by physiochemical values 201a-1 to 201a-n, and generates a plurality of targets represented by target physiochemical values 201b-1 to 201b-m. In this case, the molecule drugs are mAbs and the targets are Ags. The molecule-target generation unit 201 is configured to generate a plurality of unique synthetic drug-target candidate pairs with varying physiochemical properties. Once generated, each of the drug-target candidate pairs are applied to a PBPK computation unit 202 in which a PBPK model 202a is configured to generate PK values such as PK endpoints and profiles 202b and a target engagement/occupancy percentage value 202c for one or more scenarios of interest such as, for example, one or more dosage regimens and/or target 202d (e.g., one or more fixed doses, one or more fixed dosing schemes, fixed charge, etc.). The ML classification unit 203 is configured to either train one or more ML models 203b corresponding to one or more dosage regimens or scenarios 202d according to a one or more classification desired target occupancy conditions 203a. In this example, the ML model 203b is an interpretable decision-tree based ML model 203b trained by a decision-tree based ML algorithm in relation to a desired target occupancy condition 203a related to classifying each candidate molecule drug based on whether the target engagement/occupancy is greater than or less than a target occupancy threshold (e.g., TO%>90% or $TO\% \leq 90\%$; or TO%>99% or $TO\% \leq 99\%$). The decision-tree based ML model 203b is a classifier. The resulting interpretable decision-tree based ML model 203b is configured to classify whether data representative of physiochemical and PK values of one or more the molecule drugs satisfy the desired target occupancy conditions 203a for a particular dosage regime/scenario 202d. The drug-target physiochemical properties are selected such as, for example, binding affinity, baseline concentration, target half-life, dose, dosing scheme, and antibody charge to assist in identifying the combination of physiochemical properties of a molecule drug that are most likely to demonstrate the desired target engagement response/target occupancy.

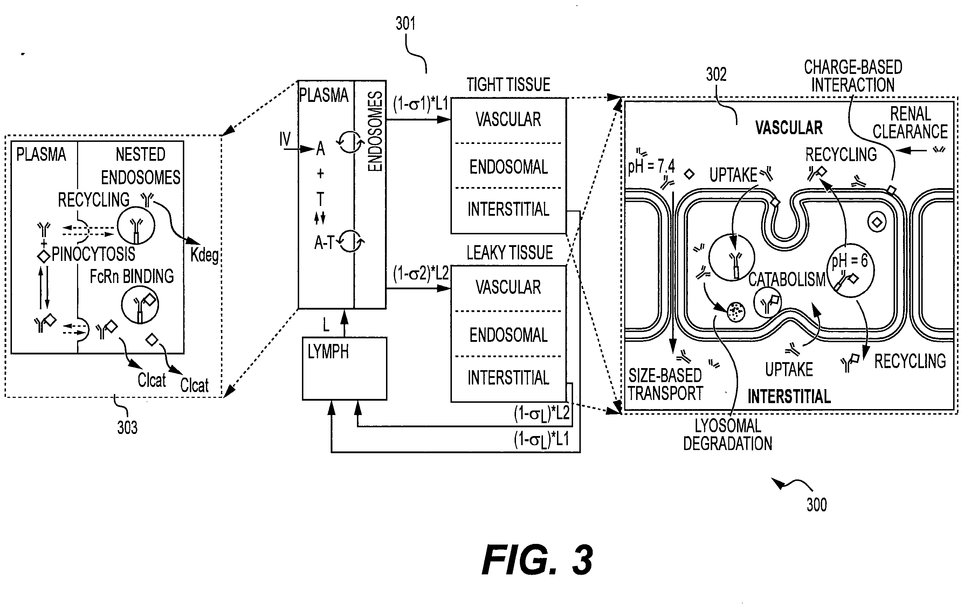

In operation, the molecule-target generation unit 201 generates a plurality of synthetic candidate pairs of large molecule drugs 201a-1 to 201a-n and their specific targets 201b-1 to 201b-m by varying a set of physiochemical properties like dose, charge, target binding affinity, target baseline, and target half-life to cover corresponding selected or desired ranges. Each virtual candidate pair 201a-1, 201b-1 signifies a unique drug-target interaction. The PBPK computation unit 202 uses a PBPK model 202a (e.g., an example mPBPK model is described in figure 3) to simulate and thus compute the PK profile and PK endpoints 202b for each virtual candidate pair, and pharmacological/pharmacodynamic effects for use in computing target engagement response represented by a target occupancy percentage value (TO%) for each corresponding selected scenario or dosage regimen 203d. The magnitude of target engagement response for each virtual candidate pair is assessed by calculating the target occupancy percentage (TO%). The PBPK model 202a (or mPBPK model) can simulate and predict the antibody PK profile, PK endpoints and TO%, where TO% is calculated as the ratio of bound target and total target in plasma, tight tissues, and leaky tissues as described by the following equation:

$$TO_p\,(\%) = \frac{C_{p,ATC}}{C_{p,ATC} + C_{p,T}},$$

where TO (%) is the target occupancy (%) in plasma, $C_{ATC}$ is concentration of drug-target complex in plasma and $C_{p,T}$ is the concentration of target in plasma. Although the above equation described a particular calculation for TO%, this is by way of example only and the invention is not so limited, it is to be appreciated by the skilled person that the PBPK model 202a may be further modified to calculate TO% using, where suitable, different parameters, values and/or approximations and the like.

A supervised tree-based machine learning algorithm is used to train the tree-based ML model 203b to classify each virtual candidate (or drug-target interaction) into an optimal or non-optimal interaction based on the calculated TO% used to annotate each training data instance in relation to the desired target occupancy conditions (e.g., a first label is assigned to a training data instance if the corresponding TO% > 90% or second label is assigned to a training data instance if the corresponding TO% <=90%). In this case, the target condition for the classifier is whether the molecule drug associated with a target has a TO% > 90% or a TO% <=90%. The tree-based ML model 203b helps to infer the optimal combination of drug and target properties that is more likely to provide the desired PK/TO endpoints and eventually the desired pharmacological effect.

As described above, many synthetic candidate pairs of large molecule drugs and their specific targets can be generated by varying properties like dose, charge, target binding affinity, target baseline, and target half-life can be generated using the molecule-target generation unit 201. Each virtual synthetic candidate signifies a unique drug-target interaction. The mPBPK model 202a is configured to simulate the PK profile and PK endpoints for each virtual synthetic candidate pair. The magnitude of target engagement response for each virtual candidate is assessed by calculating the target occupancy percentage (TO%) from the PK profiles and PK endpoints resulting from the mPBPK model simulations. The tree-based ML model 203b when trained with supervised learning is used to classify each virtual candidate (or drug-target interaction) into an optimal or non-optimal interaction based on the calculated TO% as described herein. The ML model 203b helps to infer the optimal combination of drug and target properties that is more likely to provide the desired PK/TO endpoint and eventually desired pharmacological effect.

The synthetic data is used to train an ML model 203a classifier for each of different dose variations, dosing frequencies, antibody charge variations, and across different forms of receptors (soluble or membrane-bound). This may be used to form an ensemble of ML models for classifying drug-target candidate pairs in relation to one or more different dose variations, dosing frequencies, antibody charge variations, and across different forms of receptors and the like.

As an example, a list of scenarios for generating data for development of the ML model is represented as a list of mPBPK model simulations to obtain PK endpoints for synthetic candidates (n = 10000) in the following table:

| Simulation ID | Charge | Dose (mg/kg) | Regimen | Receptor Form | Criterion |
|---|---|---|---|---|---|
| 1 | 0 | 0.1 | Bolus (IV) | Soluble | $TO_{max}$ |
| 2 | +5 | 0.1 | Bolus (IV) | Soluble | $TO_{max}$ |
| 3 | -5 | 0.1 | Bolus (IV) | Soluble | $TO_{max}$ |
| 4 | 0 | 0.1 | Bolus (IV) | Membrane | $TO_{max}$ |
| 5 | +5 | 0.1 | Bolus (IV) | Membrane | $TO_{max}$ |
| 6 | -5 | 0.1 | Bolus (IV) | Membrane | $TO_{max}$ |
| 7 | 0 | 1 | Bolus (IV) | Soluble | $TO_{max}$ |
| 8 | +5 | 1 | Bolus (IV) | Soluble | $TO_{max}$ |
| 9 | -5 | 1 | Bolus (IV) | Soluble | $TO_{max}$ |
| 10 | 0 | 1 | Bolus (IV) | Membrane | $TO_{max}$ |
| 11 | +5 | 1 | Bolus (IV) | Membrane | $TO_{max}$ |
| 12 | -5 | 1 | Bolus (IV) | Membrane | $TO_{max}$ |
| 13 | 0 | 10 | Bolus (IV) | Soluble | $TO_{max}$ |
| 14 | +5 | 10 | Bolus (IV) | Soluble | $TO_{max}$ |
| 15 | -5 | 10 | Bolus (IV) | Soluble | $TO_{max}$ |
| 16 | 0 | 10 | Bolus (IV) | Membrane | $TO_{max}$ |
| 17 | +5 | 10 | Bolus (IV) | Membrane | $TO_{max}$ |
| 18 | -5 | 10 | Bolus (IV) | Membrane | $TO_{max}$ |
| 19 | 0 | 0.1 | Q2W (IV) | Soluble | $TO_{min}$ |
| 20 | 0 | 1 | Q2W (IV) | Soluble | $TO_{min}$ |
| 21 | 0 | 10 | Q2W (IV) | Soluble | $TO_{min}$ |
| 22 | 0 | 1 | Q2W (IV) | Membrane | $TO_{min}$ |
| 23 | +5 | 1 | Q2W (IV) | Soluble | $TO_{min}$ |

(continued)

| Simulation ID | Charge | Dose (mg/kg) | Regimen | Receptor Form | Criterion |
|---|---|---|---|---|---|
| 24 | -5 | 1 | Q2W (IV) | Soluble | $TO_{min}$ |
| 25 | 0 | 1 | Q1W (IV) | Soluble | $TO_{min}$ |
| 26 | 0 | 1 | Q4W (IV) | Soluble | $TO_{min}$ |

In the above table, the Charge column represents the net surface charge on antibody candidate; the Dose column represents administered antibody dose; the Regimen column represents the dosing scheme, where IV refers to intravenous, Q1W refers to dose administered every 1 week, Q2W refers to dose administered every 2 weeks, and Q4W refers to dose administered every 4 weeks; the Criterion column represents the PK endpoint used for ML classification, where $TO_{max}$ is the target occupancy (TO) % calculated at maximum drug concentration, $TO_{min}$: target occupancy (TO) % calculated at minimum drug concentration. The synthetic pairs of molecules along with the PK endpoints including target occupancy (TO%) can be used to generate a labelled training dataset for use in training an ML model for each of the different scenarios, which may be combined to form an ensemble ML model for classifying unknown candidate molecule-target pairs (e.g., the synthetic molecule-target pairs or real-world molecule-target pairs and the like).

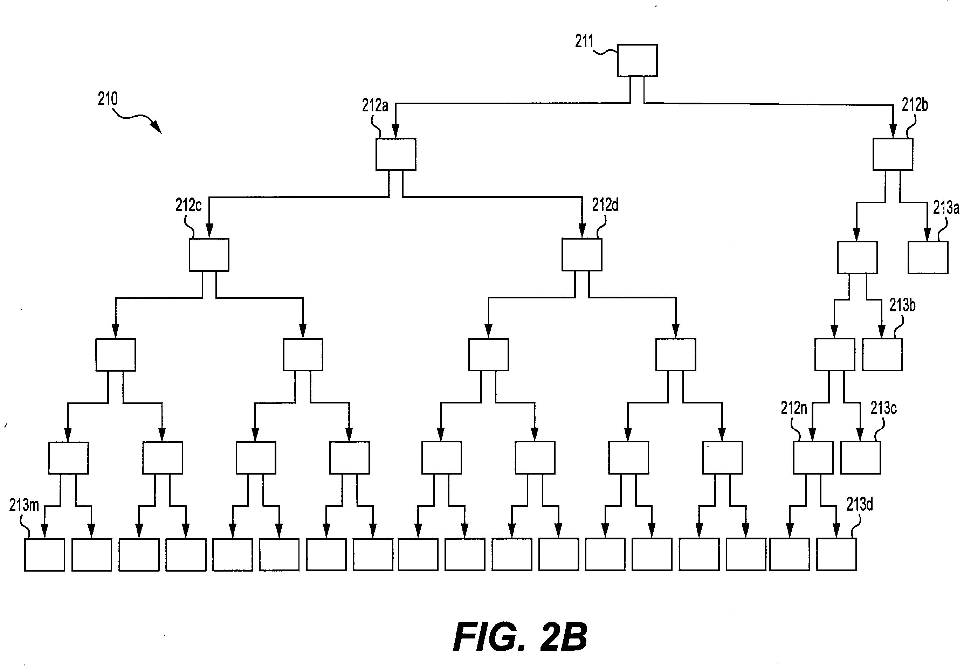

Figure 2b illustrates an example trained tree-based ML model 210 based on the interpretable decision-tree based ML model 203b, where the tree-based ML model 210 includes a root node 211 coupled via edges to a plurality of decision nodes 212a-212n and a plurality of terminal nodes 213a-213m used by the trained tree-based ML model 210 for classifying one or more molecule drugs associated with a target for a dosage regimen. In an example, an ensemble of tree-based ML models may be used to classify one or more molecule drugs associated with a target for multiple dosage regimen, each tree-based ML model in the ensemble being associated with a particular dosage regimen of the multiple dosage regimens.

Referring to figures 2a and 2b, the synthetic molecule-target pairs 201a-1 to 201a-n and 201b-1 to 201b-m and the corresponding sets of PK values 202b and target engagement response/target occupancy values 202c for various dosage regimes 202d can be used to form a labelled training dataset for training the ML model for different dose variations, dosing frequencies, antibody charge variations, and across different forms of receptors (soluble or membrane-bound). For example, a portion of a labelled training dataset is shown below in the Example ML Training Dataset table in which only a subset of molecule drug and target pairs (e.g., only 5 pairs) are shown in which each row represents a different molecule drug and target pair. In this example, only a subset of the physiochemical values for the molecule drug and target pair are illustrated and these include, without limitation, for example target baseline ($T_0$), target half-life ($t_{1/2}$), and drug's binding constant ($K_D$). As well, each row is also annotated/labelled (e.g., '1', '0') based on comparing the target occupancy value of the molecule drug and target pair with two desired target occupancy conditions (e.g., TO%≤90% and TO%>90%). The labels used to annotate each row is a '1' when the target occupancy of the molecule drug and target pair is greater than 90%, and a '0' when the target occupancy of the molecule drug and target pair less than or equal to 90%.

**Example ML Training Dataset table:**

| ID | Target baseline ($T_0$) [nM] | Target half-life ($t_{1/2}$) [hr] | Drug's binding constant ($K_D$) [nM] | Target Occupancy (TO) [1: >90%, 0: ≤ 90%] |
|---|---|---|---|---|
| 1 | 2.42 | 16.89 | 0.005 | 1 |
| 2 | 496.86 | 0.07 | 0.11 | 0 |
| 3 | 55.77 | 200.50 | 15.34 | 0 |
| 4 | 297.76 | 0.96 | 1.27 | 0 |
| 5 | 4.02 | 5.08 | 0.005 | 1 |

Thus, a labelled training dataset for use in training an ML model for each of the different scenarios, which may be combined to form an ensemble ML model for classifying unknown candidate molecule-target pairs (e.g., the synthetic molecule-target pairs or real-world molecule-target pairs and the like).

In order to generate the trained tree-based ML model 210 for a particular scenario (e.g., a particular dosage regimen), a tree-based ML algorithm is used to train model parameters of the ML model using the labelled training dataset generated in relation to the particular scenario. For example, when trained using an example 10000 synthetic drug-target candidate pairs, where the antibody was modelled by the mPBPK model 203a as being administered via an intravenous (IV) route at a dosage scenario of 1 mg/kg once every 2 weeks (Q2W) and bound to only soluble forms of receptors. The decision-tree based model 203b/210 uses an appropriate cut-off value for each input feature ($K_D$ (or KD), $T_0$, $t_{1/2}$) associated with a drug-target candidate into an optimal class (e.g., TO%>90%) or non-optimal class (e.g., TO%≤90%) based on the TO% value observed at minimum concentration in plasma at steady state. In this example, although the input features or properties of a drug-target candidate pair include a binding constant value ($K_D$ or KD), target baseline ($T_0$), and target half-life ($t_{1/2}$), this is by way of example only and these need not be so limited, it is to be appreciated by the skilled person that any set of input features or physiochemical properties associated with a drug-target candidate may be considered including, without limitation, MW, antibody charge (Q), and binding constant value (KD), a target half-life ($t_{1/2}$) and target baseline ($T_0$) and the like and/or any other suitable physiochemical properties as the application demands.

In this case, the decision-tree based model 203b/210 is trained using the example 10000 synthetic drug-target candidate pairs based on input features ($K_D$ (or KD), $T_0$, $t_{1/2}$), in which the antibody was modelled as being administered via an intravenous (IV) route at a dosage scenario of 1 mg/kg once every 2 weeks (Q2W) and bound to only soluble forms of receptors. The decision tree model 203b/210 for binary classification was built using a labelled training dataset based on the example 10000 synthetic drug-target candidate pairs. In this example, the decision tree algorithm used was based on, without limitation, for example the sklearn package in Python using default hyperparameters, except *max_depth = 5, criterion = 'gini', min_samples_split =3, splitter = 'best', class_weight = 'balanced'*. The resulting trained decision-tree based model 203b/210 comprises a set of ML-derived rules resulting from training the decision tree algorithm using a labelled training dataset based on the example 10000 synthetic drug-target candidate pairs, which are annotated based on a desired one or more TO% condition(s) or other desired conditions associated with pharmacological/ pharmacodynamic effects. In this case, the trained decision-tree based model 203b/210 is configured to classify drug-target candidates from applying their input features ($K_D$ (or KD), $T_0$, $t_{1/2}$) and output a classification for the optimal class (e.g., TO%>90%) or the non-optimal class (e.g., TO%≤90%) based on the following ML-derived rules generated using the decision tree algorithm:

if (KD <= 8.965) and ($T_0$ <= 4.047) and ($T_0$ <= 2.261) and (KD <= 8.503) and (KD <= 7.943) then class: optimal (proba: 99.94%) | based on 3,605 samples;

if (KD > 8.965) and (KD > 8.988) then class: non-optimal (proba: 100.0%) | based on 2,481 samples;

if (KD <= 8.965) and ($T_0$ > 4.047) and ($t_{1/2}$ <= 19.916) and ($T_0$ > 9.114) and ($t_{1/2}$ <= 14.208) then class: non-optimal (proba: 100.0%) | based on 1,152 samples;

if (KD <= 8.965) and ($T_0$ > 4.047) and ($t_{1/2}$ > 19.916) and ($T_0$ <= 92.58) and ($t_{1/2}$ > 40.74) then class: optimal (proba: 96.02%) | based on 251 samples;

if (KD <= 8.965) and ($T_0$ > 4.047) and ($t_{1/2}$ <= 19.916) and ($T_0$ <= 9.114) and ($t_{1/2}$ <= 5.457) then class: non-optimal (proba: 97.14%) | based on 175 samples;

if (KD <= 8.965) and ($T_0$ > 4.047) and ($t_{1/2}$ > 19.916) and ($T_0$ > 92.58) and ($t_{1/2}$ <= 137.423) then class: non-optimal (proba: 100.0%) | based on 151 samples;

if (KD <= 8.965) and ($T_0$ <= 4.047) and ($T_0$ > 2.261) and ($t_{1/2}$ > 2.182) and (KD <= 7.626) then class: optimal (proba: 100.0%) | based on 136 samples

if (KD <= 8.965) and ($T_0$ > 4.047) and ($t_{1/2}$ > 19.916) and ($T_0$ <= 92.58) and ($t_{1/2}$ <= 40.74) then class: optimal (proba: 68.76%) | based on 80 samples;

if (KD <= 8.965) and ($T_0$ > 4.047) and ($t_{1/2}$ <= 19.916) and ($T_0$ > 9.114) and ($t_{1/2}$ > 14.208) then class: non-optimal (proba: 94.12%) | based on 68 samples;

if (KD <= 8.965) and ($T_0$ <= 4.047) and (T0 > 2.261) and ($t_{1/2}$ <= 2.182) and ($T_0$ > 2.628) then class: non-optimal (proba: 88.23%) | based on 68 samples;

if (KD <= 8.965) and ($T_0$ > 4.047) and ($t_{1/2}$ > 19.916) and ($T_0$> 92.58) and ($t_{1/2}$ > 137.423) then class: non-optimal (proba: 64.99%) | based on 60 samples;

if (KD <= 8.965) and ($T_0$ <= 4.047) and ($T_0$ > 2.261) and ($t_{1/2}$ <= 2.182) and ($T_0$ <= 2.628) then class: optimal (proba: 64.45%) | based on 45 samples;

if (KD <= 8.965) and ($T_0$ > 4.047) and ($t_{1/2}$ <= 19.916) and ($T_0$ <= 9.114) and ($t_{1/2}$ > 5.457) then class: optimal (proba: 90.7%) | based on 43 samples;

if (KD <= 8.965) and ($T_0$ <= 4.047) and ($T_0$ <= 2.261) and (KD <= 8.503) and (KD > 7.943) then class: optimal (proba: 92.0%) | based on 25 samples;

if (KD <= 8.965) and ($T_0$ <= 4.047) and ($T_0$ <= 2.261) and (KD > 8.503) and ($T_0$ <= 0.28) then class: optimal (proba: 100.0%) | based on 18 samples;

if (KD <= 8.965) and ($T_0$ <= 4.047) and ($T_0$ <= 2.261) and (KD > 8.503) and ($T_0$ > 0.28) then class: non-optimal (proba: 100.0%) | based on 6 samples;

if (KD <= 8.965) and ($T_0$ <= 4.047) and ($T_0$ > 2.261) and ($t_{1/2}$ > 2.182) and (KD > 7.626) then class: optimal (proba: 50.01%) | based on 2 samples;

Although the example rules for the trained decision-tree based model 203b/210 are described above, this is by way of example only and the trained decision-tree based model 203b/210 is not so limited, it is to be appreciated by the skilled person that the ML-derived rules of a trained decision-tree based model will change depending on, without limitation, for example the types of molecules and their physiochemical properties, targets and their physiochemical properties, PK values associated with molecule drug-target pairs, pharmacological effects/pharmacodynamic values associated with the molecule drug-target pairs, desired conditions associated with pharmacological/pharmacodynamic effects, type of PBPK models, dosage regimens and/or other scenarios, the training datasets, the number of training data instances, the model hyperparameters, the type of ML model algorithm and/or the like, combinations thereof, modifications thereto, and/or as the application demands.

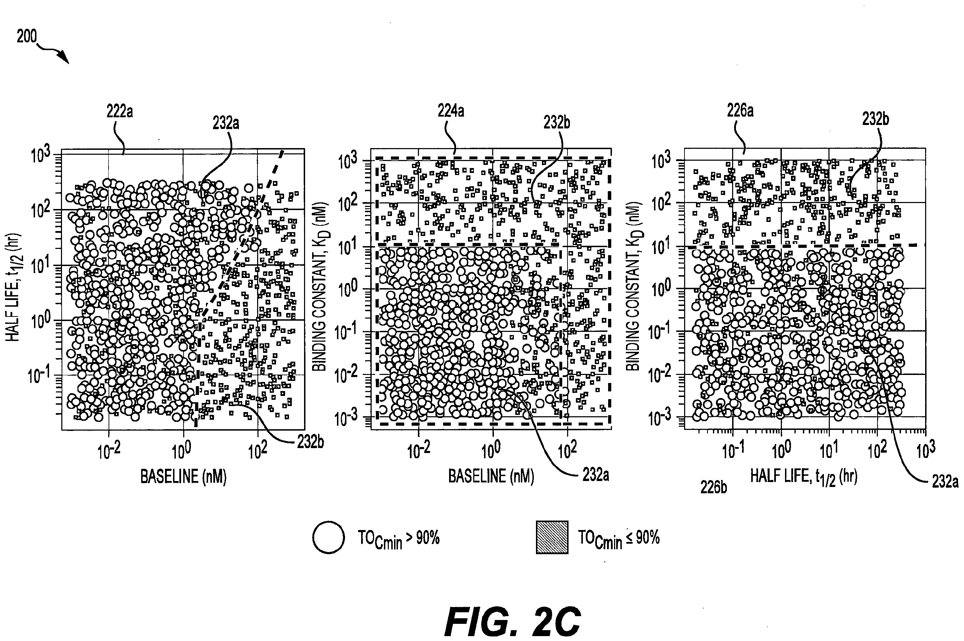

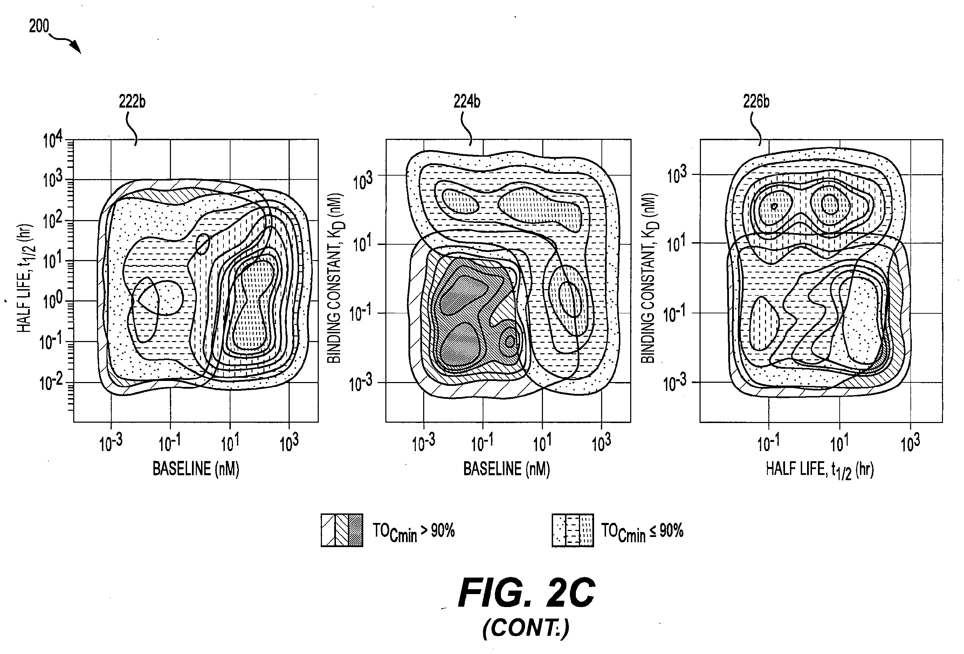

Figure 2c illustrates example set of plots 220 using the decision-tree based model 203b or 210 of figures 2a and/or 2b when trained using 10000 synthetic drug-target candidate pairs, where the antibody was modelled as being administered via an intravenous (IV) route at 1 mg/kg once every 2 weeks (Q2W) and bound to only soluble forms of receptors. The decision-tree based model 203b uses an appropriate cut-off value for each input feature ($K_D$, $T_{s0}$, $t_{1/2s}$) to classify each virtual drug-target candidate into an optimal (large circles in plots 222a, 224a, 226a) or non-optimal (small circles in plots 222a, 224a, 226a) class based on the TO% value observed at minimum concentration in plasma at steady state. As illustrated by the plots 222a-226a and 222b-226b in figure 2c, the decision-tree based model 203b indicates that a drug administered at 1 mg/kg once every two weeks should have greater than 90% target engagement/occupancy when the binding constant ($K_D$) is below 9 nM for a class of soluble targets that have baseline values below 4 nM in plasma. In this example, the tree-based rules for optimal class prediction were chosen based on Gini impurity close to 0 and accuracy close to 100%. It is important to choose the best rule with the highest accuracy as it increases the likelihood for true prediction for other unseen datasets. In this scenario, $K_D$ and $T_0$ (i.e., binding constant value and target baseline) are the most important physiochemical properties to predict more than 90% target occupancy. The physiochemical parameter $t_{1/2}$ (i.e., target half-life) is illustrated to have the least influence in decision making in this scenario. However, the class of soluble receptors with low half-life and high baseline may be the least ideal combination of target properties to achieve greater than 90% target occupancy in plasma. The binary classification tree of the trained decision-tree based model 203b for the above scenario is provided in figure 2b. Each tree-based model was evaluated on a separate validation data set based on the following evaluation metrics such as classification metric, confusion matrix, and area under the receiver-operating curve (ROC).

The below tables also illustrate an evaluation of the tree-based ML model performance across various different types of tree-based ML algorithms, which in this example include decision tree, random forest, gradient boosting ML classifier algorithms. In these examples, the decision trees were each trained for the same scenario (e.g., as indicated above in figure 2c). The decision tree model for binary classification was built using the sklearn package in Python using default hyperparameters, except *max_depth = 5, criterion = 'gini', min_samples_split =3, splitter = 'best', class_weight = 'balanced'.* Although a specific set of hyperparameters are described here, this is by way of example only and the invention is not so limited, it is to be appreciated by the skilled person that different hyperparameters may be selected, set and/or used for training a tree-based ML model for binary and/or multi-class classifications and/or the same or other types of scenarios and datasets as the application demands. Although tree-based ML model is described, this is by way of example only and the invention is not so limited, it is to be appreciated by the skilled person that any other suitable ML algorithm may be used to train a suitable ML model for binary and/or multi-class classification for the same of various other scenarios and/or datasets as the application demands.

Training performance metrics, shown below in the Example Training Metric table, include accuracy, precision, F1 score are mean % for 5-fold cross validation when training each algorithm. The standard deviation (SD) for each fold cross-validation is provided in braces. The Example Testing Metric table includes testing metrics of accuracy, precision, F1-score for evaluating model performance on a test dataset.

**Example Training Metric table**

| Algorithm | Accuracy (SD) | Precision (SD) | F1-score (SD) | Time (s) |
|---|---|---|---|---|
| Decision Trees | 98.8 (0.002) | 98.3 (0.0017) | 98.8 (0.002) | 0.0330 |
| Random Forest | 98.9 (0.002) | 98.7 (0.0019) | 98.8 (0.002) | 1.3069 |
| Gradient Boosting | 99.2 (0.0018) | 98.9 (0.003) | 99.2 (0.002) | 2.4278 |

**Example Testing Metric table**

| Algorithm | Accuracy (SD) | Precision (SD) | F1-score (SD) |
|---|---|---|---|
| Decision Trees | 98.7 | 98.7 | 98.4 |
| Random Forest | 97 | 97.1 | 95.1 |
| Gradient Boosting | 99 | 99 | 98.9 |

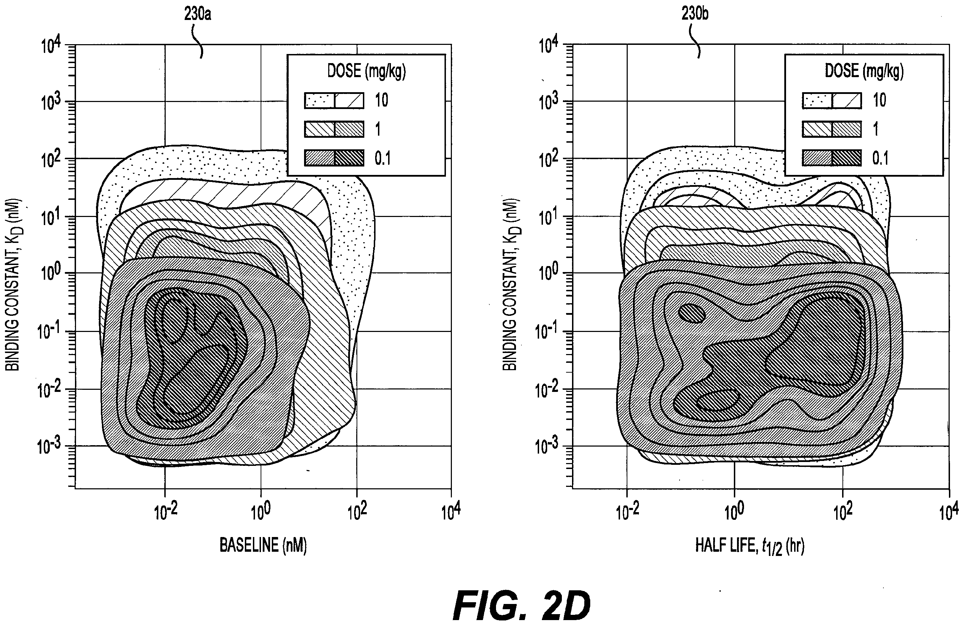

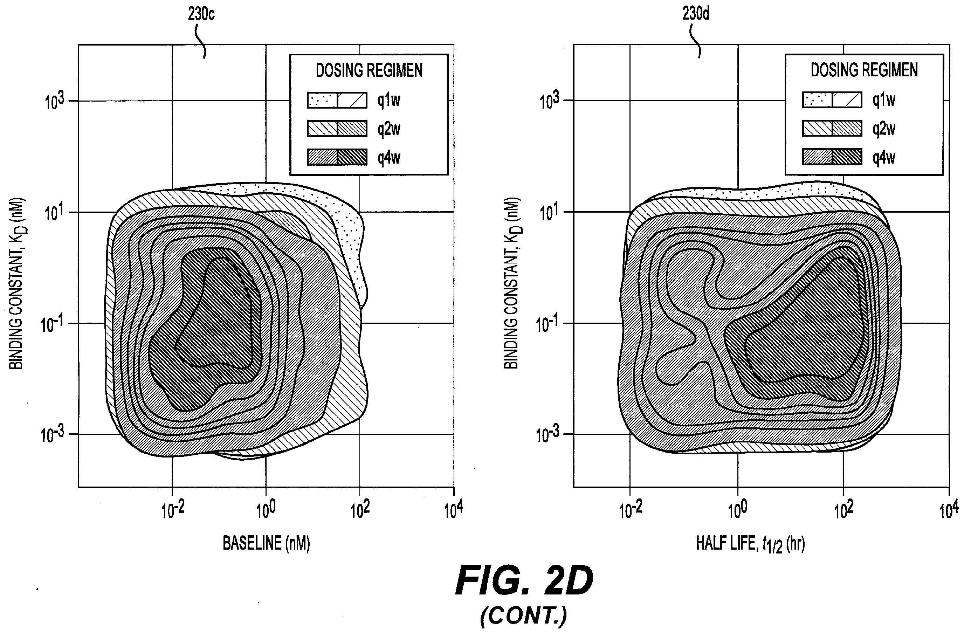

Figure 2d illustrates four plots 230a, 230b, 230c, and 230d illustrating optimal drug-target properties for target engagement for different scenarios in relation to doses and dosing regimens. In this example, a separate tree-based ML model (e.g., decision tree) was trained for each scenario based on the tree-based ML model as described with reference to any of Figures 1a to 2c. A separate tree-based ML model was trained and validated for synthetic data for each different scenario including different doses of 0.1, 1, and 10 mg/kg administered once every 2 weeks (Q2W) resulting in plots 230a and 230b. A separate tree-based ML model was also trained and validated for synthetic data for each different scenario based on a fixed dose of 1 mg/kg administered once every week (Q1W), once every two weeks (Q2W), and once every 4 weeks (Q4W) with the results illustrated in plots 230c and 230d. In both cases, only optimal properties dependent on the condition that target occupancy > 90% at minimum plasma concentration at steady state are shown.

These examples illustrate the difference in optimal rules and values of drug-target properties for different doses and dosing regimens/schemes. In plots 230a and 230b, the corresponding trained decision tree-based ML models for each scenario provided an optimal region for drug-target binding constant ($K_D$), target baseline, and target half-life values for bolus and repeated IV dose of 0.1, 1, and 10 mg/kg. The optimal rule for low dose (0.1 mg/kg) scenario suggests a $K_D$ value below 1 nM as illustrated in plots 230a and 230b. A drug administered at such low dose may achieve 90% target occupancy with class of soluble receptors with baseline values below 2 nM. As illustrated in plots 230a and 230b, as the dose is increased from 0.1 mg/kg to 1 mg/kg, the cut-off for optimal $K_D$ value increases to 9 nM as well as target baseline values increases to 4 nM. Similarly, as illustrated in plots 230a and 230b, at high dose of 10 mg/kg, the optimal cut-off for $K_D$ increased to 82 nM. At lower administered dose the drug clears faster and undergoes target-mediated disposition. Therefore, to achieve greater than 90% target occupancy at such a low dose, drug-target affinity should be higher ($K_D$ values should be lower). The optimal rules suggested that $K_D$ and target baseline were relatively more important to classify a given drug-target pair based on target engagement response. Target baseline concentration and target half-life are relevant target properties that govern drug-target interaction and desired target pharmacology. However, to achieve more than 90% target occupancy at different doses, target half-life seems to be the least influential in the decision-making process.

Referring to plots 230c and 230d, the effect of dosing scheme on optimal TO% is observed when drug is administered at a repeated dose of 1 mg/kg once every 1 week (Q1W), once every 2 weeks (Q2W), and once every 4 weeks (Q4W). Again, trained decision tree-based ML models were trained for these different regimen scenarios. The resulting ML tree-based rules deemed that as dosing frequency increases, the optimal range for $K_D$, $T_{s0}$, and $t_{1/2s}$ increases. The effect of dosing frequency on optimal drug and target properties can be observed in the density plots 230c and 230d. For Q4W, Q2W, and Q1W dosing scheme, $K_D$ values below 4.25, 9, and 15, can be observed respectively. As can be seen, $K_D$ value around 4 nM will provide greater than 90% target occupancy for a given class of soluble receptors with baseline values below 2, 4, and 8.3 nM, respectively. As can be seen in plot 230d, target half-life has the least dependence on dosing scheme / regimen scenario and was insufficient in solely determining the optimal class.

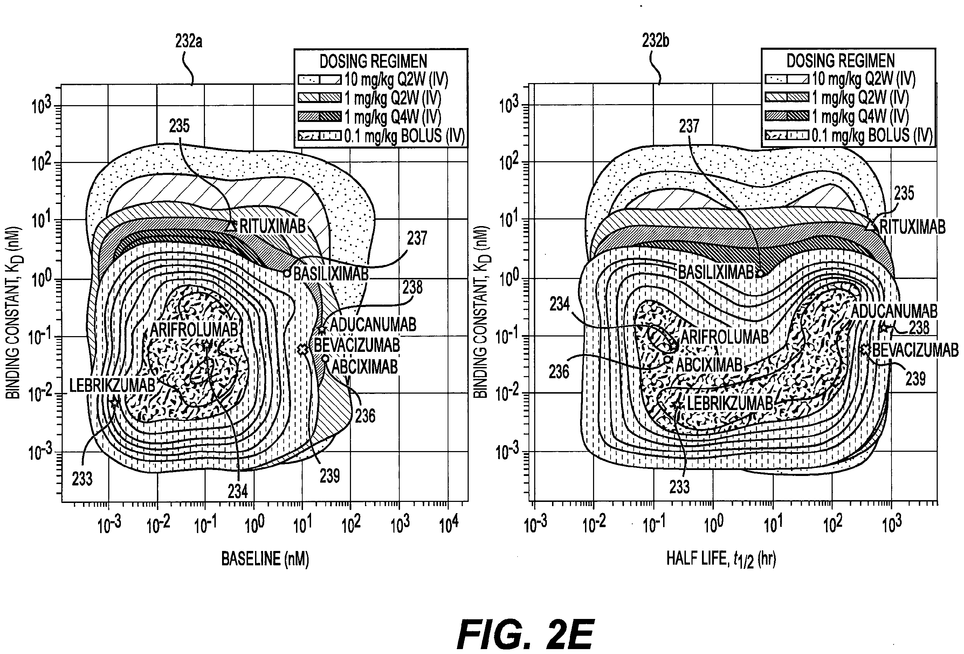

Figure 2e illustrates two plots 232a and 232b used to validate the ML-derived rules of the trained tree-based ML model 203b/210 as described with reference to Figures 2b and 2c. The ML-derived rules were validated in a study using real-world compound / molecule data for clinically approved monoclonal antibodies, which may be classified as large molecules. A list of literature-derived properties of the approved monoclonal antibodies and their respective targets, as well as recommended clinical dose and regimen used in the study are provided in the following table:

**Experimental data for approved monospecific antibodies.**

| Compound | Target | Binding constant [nM] | Target Baseline [nM] | Target Half Life [hr] | Clinical Dose / Regimen (Route) | Assumption* (Dose / Regimen) |
|---|---|---|---|---|---|---|
| Lebrikizumab | IL-13 | 0.0068 | 0.0013 | 0.25 | 1.8 mg/kg Q4W (IV) | 1 Q4W |
| Anifrolumab | IFNAR | 0.067 | 0.0999 | 0.21 | 300 mg Q4W (IV) | 1 Q4W |
| Rituximab | CD20 | 8 | 0.320 | 426 | 1000 mg (IV) Q2W | 10 Q2W |
| Abciximab | GPIIb/ IIIa | 0.0411 | 29.8 | 0.18 | 0.25 mg/kg (IV) bolus | 0.1 bolus |
| Basiliximab | CD25 | 1.2 | 4.8 | 6.39 | 20 mg (IV) bolus 150 mg (IV) | 0.1 bolus |
| Aducanumab | tau VEGF | 0.131 | 27 | 736.8 | Q4W 5 mg/kg (IV) | 1 Q4W |
| Bevacizumab | -A | 0.058 | 11.3 | 345 | Q2W | 1 Q2W |

*For simplicity, dose assumptions are made to match the dose and regimen to 0.1, 1 and 10mg/kg as was used for the trained ML model 203b/210.

In this study, the trained ML model 203b/210 can make predictions for a fixed dose and regimen administered intravenously. Considering this limitation of the ML model 203b/210, some necessary but reasonable assumptions were made to validate the ML model 203b/210 against real-world data. For example, rather than directly using the recommended clinical doses described in the above table, the recommended clinical dose for each antibody were matched (e.g., rounded) to the closest dose from the selection of 0.1, 1, or 10 mg/kg. For example, the clinical dose for Lebrikizumab is 1.8mg/kg, which means the closest dose from 0.1, 1.0 or 10mg/kg is 1.0mg/kg. Similarly, the clinical dose for Basiliximab is 20mg/kg, which means the closest dose from 0.1, 1.0 or 10mg/kg is 10mg/kg. For each dose/regimen scenario, the trained ML model 203b/210 was used to predict the optimal $K_D$, $T_0$, and $t_{1/2}$ values for each antibody at the assumed dose and recommended regimen administered intravenously. The optimal drug-target properties were validated for the following scenarios, 0.1 mg/kg bolus, 1 mg/kg Q4W, 1 mg/kg Q2W, and 10 mg/kg Q2W as illustrated in plots 232a and 232b.

The real-world compounds /molecules of Lebrikizumab 233, Anifrolumab 234, Rituximab 235, Abciximab 236, Basiliximab 237, Aducanumab 238, and Bevacizumabare 239 are plotted on plots 232a and 232b. The experimental properties of most antibodies lie within the predicted optimal boundaries of drug-target properties when administered at their respective dose and dose regimen. As illustrated in plot 232a, most of the real-world compounds / molecules 233, 234, 235, 237, 238, and 239 have target $K_D$ and $T_0$ values that lie within the predicted optimal bounds except for Abciximab 236, in which its target, $K_D$ and $T_0$ values lie outside the predicted optimal bounds. For Abciximab 236, this discrepancy may be because the assumed dose is lower than the clinical dose (0.25 mg/kg bolus). It is likely that the optimal values of $K_D$ and $T_0$ will be higher for a dose greater than 0.1 mg/kg, as seen from the dose-dependence in **Error! Reference source not found..** Moreover, as illustrated in plot 232b, the optimal $t_{1/2}$ values for each scenario were validated very well against the experimental observation for each of the antibodies 233 to 239. These preliminary real-world validations provide the advantage of validating the accuracy of the ML-derived rules according to embodiments in which more compound / molecule data will assist to increase confidence in ML predictions.

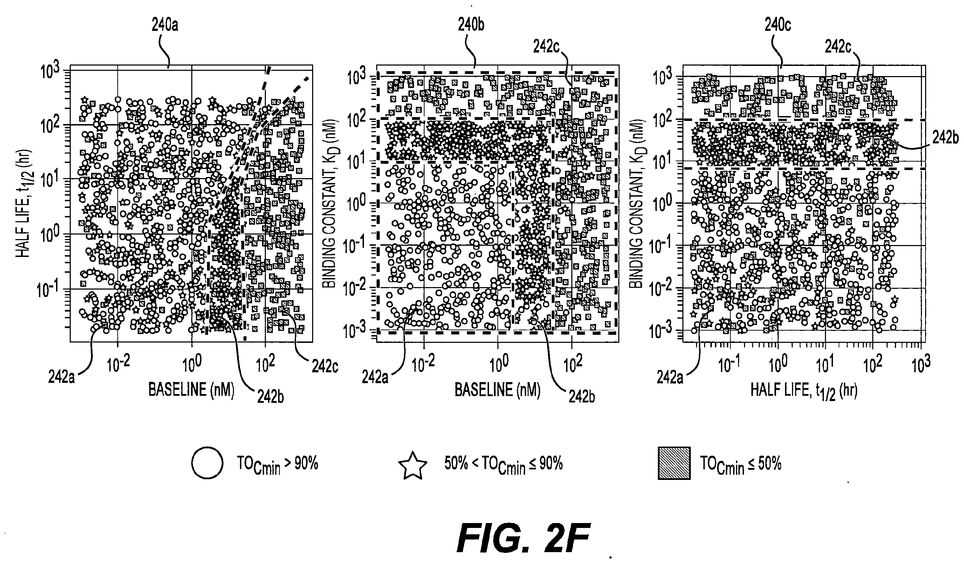

Figure 2f illustrates resulting pairwise scatter plots 240a, 240b, and 240c for a tree-based ML model 203b that was trained to perform multi-label classification, in particular, classified based on target occupancy % (TO%) < 50%, 50-90%, and >90%. In this case, the tree-based ML model 203b was trained, as described herein, using synthetic data for drug administration at 1 mg/kg once every two weeks (Q2W) to classify whether candidate molecule drugs meet one of multiple target occupancy conditions (e.g., TO%< 50%, TO% between 50-90%, TO% > 90%). In this case, the ML model 203b was trained to classify the candidate molecule drug data into three classes based on TO% at minimum concentration in plasma at steady state. The result plots 240a, 240b, and 240c of figure 2f illustrate optimal regions for selection of drug-target binding constant for a given target with baseline and half-life that lead to low, medium, and high target occupancy. As observed, there are clear boundaries for the three classes of high target occupancy 242a, medium target occupancy 242b, and low target occupancy 242c for separation of virtual drug-target properties for the respective classes. In this case, the trained tree-based ML model 203b had an accuracy of 99%, and class precision of 99%, 98%, and 99% for low, medium, and high classes 242c, 242b and 242a, respectively.

As can be seen in figures 2a to 2f, the decision tree-based classification models and/or other rule-based ML models provide several advantages including rapid training, efficient computational load during inference, and being explainable due to its rule-based interpretable nature; tree-based ML models are capable of identifying a relationship between input molecule drug-target pairs (e.g., molecule drug-target properties) and output target occupancy values (TO%) using recursive partitioning in a tree-like manner. This interpretable ML model type delineates the process of obtaining the predicted class from the given input, which provides an advantage over other black-box machine learning algorithms such as artificial NNs.

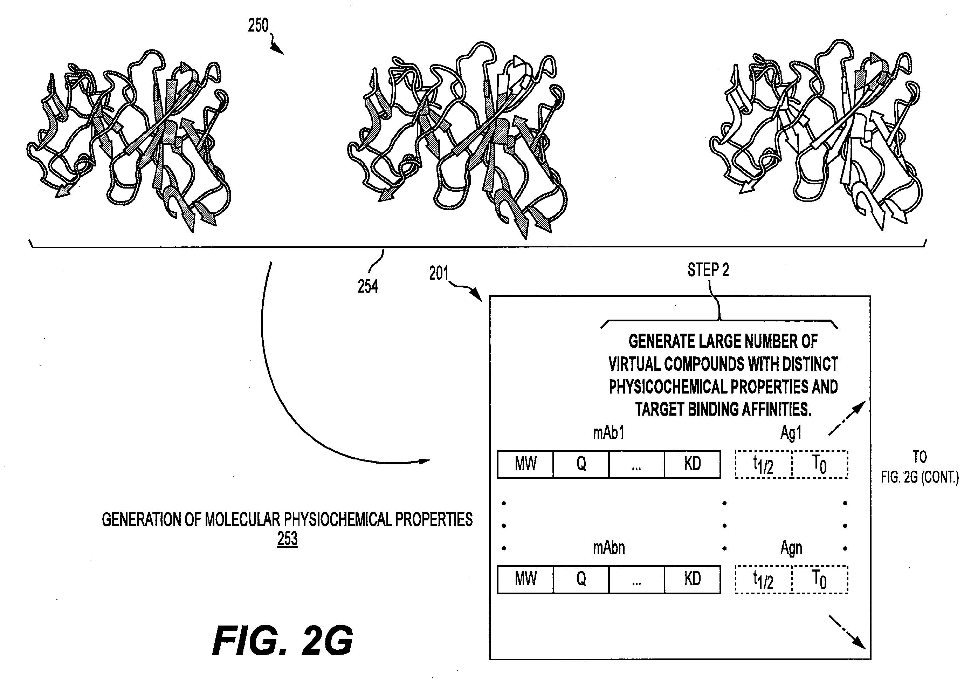

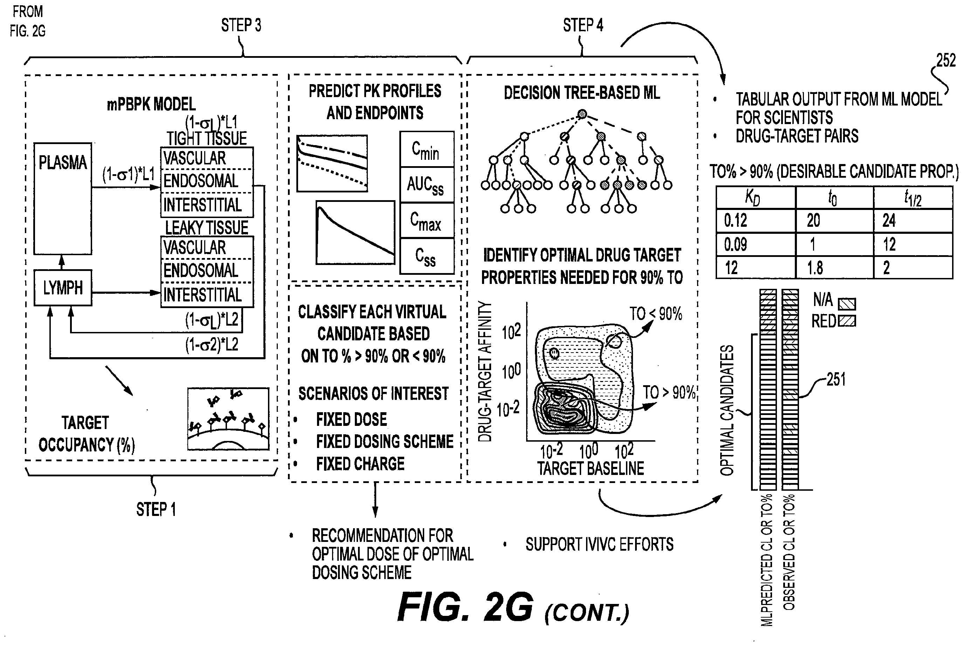

Figure 2g illustrates another example PBPKIML model system 250 based on the PBPK/ML model system 200 of figure 2a illustrating further applications for drug optimization and downstream processes. For example, the PBPK/ML model system 250 enables researchers to screen and optimize a large amount of antibody candidates based on optimal ADME, PK, and target engagement to specific antigens, and reduce attrition rate and overall discovery time for lead candidates. The PBPK/ML model system 200 and 250 provides an in silico high-throughput screening that assists in significantly speeding up the hit-to-lead rates for identifying which candidate molecule drug-target pair hits can be moved forward towards synthesis into real-world molecule drugs and subsequent downstream *in vitro* wet lab analysis and/or *in vivo* trials. Such a high-throughput screening in silico and evaluation of large molecule drug properties allow candidate evaluation against one or more pharmacological/pharmacodynamic effects such as, for example, target occupancy and engagement extremely early, which significantly narrows or reduces the candidate molecule drug/target space within the drug discovery and development pipeline. This further leads to a significant reduction in lead-time from candidate to real-world molecule drugs for treatments.

For example, some specific applications of the PBPK/ML model system 200 and/or 250 include one or more of the following:

a) Lead candidate generation, where the PBPK/ML model system 250 can be configured to determine optimal candidate properties for desired conditions associated with pharmacological/pharmacodynamic effects such as, for example, target engagement that are needed to progress to hit-to-lead stage. This *in silico* high-throughput screening technique enables scientists to narrow down and generate a shortlist 251 of the candidate molecule drugs with highest potential based on a combination of their structure-based properties, ADME, PK, and pharmacological/pharmacodynamic values (e.g., TO). This shortlist 251 may be used to automatically or semi-automatically generate or synthesize real-world molecule drugs having the properties of the candidate molecule drugs on the shortlist 251 for use in performing real-world *in vitro* wet lab analysis and/or *in vivo* trials and the like. The generated shortlist can significantly reduce the number of molecule drugs that may be experimentally evaluated in the laboratory, which further reduces development costs. For example, such shortlisting may be applied by the PBPK/ML model system 250 for medicinal chemistry where a priori knowledge of dose-driven design of antibodies is necessary. To be able to prioritize the candidate molecule drugs with the highest potential to achieve, for example, target engagement and enter lead stage at the lowest dose (or minimum dose);

b) The PBPK/ML model system 250 may be configured or adapted to provide a tabulated list 252 of combination of drug candidate properties and specific target properties to scientists for all the candidates that achieve certain desired conditions associated with corresponding pharmacological/pharmacodynamic effects such as, for example, greater than 90% target engagement/occupancy, which can be used by users (e.g., scientists) as an initial reference sheet to focus on the candidates with most potential;

c) The PBPK/ML model system 250 can be configured for use by research teams for *in vitro* ADME screening efforts during hit-to-lead or early lead optimization, visualization of the optimal spaces of candidate properties to define the most critical combination of properties for screening.

d) The PBPK/ML model system 250 can be configured to support *in vitro/in vivo* correlation (IVIVC) efforts, where the current framework provides a platform that can incorporate more *in vitro* derived properties (besides binding constant KD) such as charge, FcRn binding etc. and relate these drug properties with corresponding pharmacological/pharmacodynamic effects such as, for example, TO% but also PK-based clearance (CL). Such a property-based prediction of (human) clearance is an important endpoint during IVIVC studies to propose recommendations for halting, investigating, and/or advancing a candidate series to the next stage of a drug optimization pipeline/campaign.

e) The PBPK/ML model systems 200 or 250 can be further modified and/or configured to be integrated with surrogate ML or molecular dynamics models 253 that predict molecular properties of antibodies based on structure or sequence of the antibodies 254. Such an integration performed in the generation molecule/target unit 201 of PBPKIML model system 200 and 250 to obtain molecular properties can be used to replace and/or supplement synthetic generation of these properties.

Figure 3 illustrates an example PBPK component 300 for use with the PBPK/ML model systems 200 and 250 and/or processes 100, 105, 110, 120 and the like as described with reference to figures 1a to 2e. In this case, the PBPK component 300 uses a minimum PBPK (mPBPK) model 301, which is configured to account for the effect of antibody weight, size, charge, binding to FcRn and different forms of specific receptors in different pH environments. The mPBPK model 301 is configured to apply the two-pore hypothesis to predict size-based clearance and exposure of full-length antibodies (e.g., 150 kDa) and antibody fragments (e.g., 50-100 kDa) and quantitatively relate effect of charge on PK parameters like uptake rate, non-specific binding affinity, and volume of distribution. The mPBPK model 301 is configured to predict the target-mediated disposition of a molecule drug when compound-specific and target-specific properties are known. Such a combined effect of antibody weight, size, charge, FcRn, and antigen has not been incorporated in a single mPBPK model before. By conclusively incorporating and relating a multitude of protein's physicochemical properties to observed PK, the mPBPK model 301 provides a useful PBPK model for use in PBPK/ML model systems 200 and 230 and/or processes 100, 105, 110, 120 especially in the early stages of drug optimization development where many of these properties can be optimized to improve a molecule's PK and ultimately its efficacy.

In essence, the mPBPK model 301 consists of plasma, lymph, tight tissue, and leaky tissue compartments. Each tissue compartment is divided into vascular, endosomal, and interstitial space as shown in the right blow-up section 302. The plasma compartment is nested with endosomal space. Monoclonal antibody (A) is intravenously (IV) administered in the plasma compartment, which binds to soluble antigen (T) in the plasma compartment and tissue vasculature to form an antibody-antigen complex (A-T). mAb (A), soluble antigen (T), and complex (A-T) circulate to the nested endosomes in plasma and tissue sub-compartments. Free mAb (A) and mAb-antigen complex (A-T) pinocytose into the endosomal space and bind to FcRn (black rectangle) at an acidic pH. Unbound mAb degrades in endosomes at a rate $k_{deg}$ and unbound antigen and bound mAb-antigen complex catabolize within endosomes at clearance rate $CL_{cat}$. FcRn-bound mAb and FcRn-bound mAb-antigen complex are recycled back from the endosomal space continually. Free mAb (Y-shape) also binds to membrane-bound receptors (diamonds) and negatively charged membrane proteins (dashed line) in tissue vasculature, transports through the paracellular pores and systemically clears based on their size. Free mAb and mAb-antigen complex are transported back to the systemic circulation through the lymph compartment. A representation of nested endosomes in the plasma compartment 303 is also illustrated in Figure 3. The kinetics in the nested endosomal space is similar to tissue endosomal space. In Figure 3, $\sigma_1$, $\sigma_2$, and $\sigma_L$ are vascular reflection coefficients, and L, L1, L2 are lymph flow rates in the Lymph, tight tissue, and leaky tissue compartment, respectively.

In essence, the structure of the mPBPK model 301 remains the same across species. In an example, the species the mPBPK model 301 was tested on is mice, by way of example only but the invention is not so limited, and the skilled person would understand and adjust the mPBPK model parameters accordingly for other subjects such as, for example, humans and/or other mammals and the like. The mPBPK model equations are provided below as equations A1-A51. The model parameters for the mPBPK model 301 are loosely divided into two groups, physiology-based parameters and physicochemical or compound-based parameters. Additionally, some compound-specific parameters like molecular weight, size, and charge can be related to model parameters through derived and empirical equations. An example compiled list of physiology-based and kinetic parameters and values that may be used for the example of mice are provided as:

| Parameters | Definition | Value |
|---|---|---|
| BW | Body weight | 0.028 kg |
| $V_p$ | Volume of plasma | 0.85 ml |
| $V_L$ | Volume of lymph compartment | 1.717 ml |
| $L_1$ | Lymph flow rate in tight tissue | 2.7820E-04 L/h |
| $L_2$ | Lymph flow rate in leaky tissue | 4.6792E-04 L/h |
| L | Lymph flow rate | L1 + L2 |
| $V_e$ | Endosomal volume in systemic vascular endothelial cells. | 0.005/100*BW |
| $V_{e1}$ | Volume of endosome in tight tissues | 0.09404 ml |
| $V_{e2}$ | Volume of endosome in leaky tissues | 0.03685 ml |
| $V_{v1}$ | Volume of vascular space in tight | 0.4695 ml |
| $V_{v2}$ | Volume of vascular space in leaky | 0.3477 ml |
| $V_1$ | Volume of interstitial fluid volume in tight | 3.554 ml |
| $V_2$ | Volume of interstitial fluid volume in leaky | 1.3539 ml |
| $\sigma_1$ | Reflection coefficient | 0.9 |
| $\sigma_2$ | Reflection coefficient | 0.86 |
| FR | Fraction recycled in vascular space | 0.715 |
| $FcRn_{total}$ | Total FcRn concentration | 40 $\mu$M |
| GFR | Glomerular filtration rate | 0.0167 L/h |
| $k_{up}$, p | Uptake rate | 0.05 1/h |

(continued)

| Parameters | Definition | Value |
|---|---|---|
| $k_{up}$ | Uptake rate | 0.027 1/h |
| $CL_{rec}$ | Recycling clearance rate | 7.27E-06 |
| $k_{deg}$ | Degradation rate of free drug through catabolic pathways | 42.9 1/h |
| $CL_{cat}$ | catabolic clearance rate of drug-target complex assumed same as target | 1.295 ml/day |
| $k1_{on}$ | Association rate constant for drug-FcRn binding | 0.12 1/nM.h |
| $k1_{off}$ | Dissociation rate constant for drug-FcRn binding | 1.8 1/h |
| $k_{on}$ | Association rate constant for drug-target binding at physiological pH (7.4) | 5.9 1/nM.h |
| $k_{off}$ | Dissociation rate constant for drug-target binding at physiological pH (7.4) | 0.0508 1/h |
| $ke_{on}$ | Association rate constant for drug-target binding at endosomal pH (6) | 5.904 1/h |
| $ke_{off}$ | Dissociation rate constant for drug-target binding at endosomal pH (6) | 0.0508 1/h |
| $k_{p,T}$ | Rate of degradation of soluble target | 0.3465 1/h |
| $k_{p,Tm}$ | Rate of degradation of membrane-bound target | 0.0192 1/h |
| $ICC_{p,T}$ | Baseline concentration of soluble target | 2 ng/ml |
| $ICC_{p,Tm}$ | Baseline concentration of membrane-bound target | 80 nM |
| $k_{syn}$ | Rate of synthesis of soluble target | 0.0055 nM/h |
| $k_{syn,m}$ | Rate of synthesis of membrane-bound target | 1.53 nM/h |
| $k_{int}$ | Internalization rate of membrane-bound drug-target complex | 0.015 1/h |
| $R_{m,total}$ | Total negatively charged receptor concentration | 71.86 nM |

Note: in this example, L = L1 + L2. $CL_{rec}$ is calculated using transit time (8 min) and volume of endosomes. $k_{syn}$ = $ICC_{p,T}$ *$k_{p,T}$ for soluble receptors. $k_{syn,m}$ = $ICC_{p,Tm}$ *$k_{p,Tm}$ for membrane-bound receptors.

The above example values for the corresponding physiology-based and kinetic parameters may be calculated, measured, estimated and/or found from relevant literature studies in relation to the subject of the species of interest (e.g., in this example, the subject of the species of interest is mice) for input to the mPBPK model 301. The physiological parameters such as body weight, volumes, flow rates, FcRn concentration etc., are dependent on the species of interest and can be adjusted to any species and/or subject such as, without limitation, for example other animal and/or human subjects. The baseline concentration of soluble and membrane-bound antigens can be obtained from literature studies.

The compound-specific parameters vary with the drug's physicochemical properties. In the mPBPK model 301, MW of drug, Stoke's radius ($a_e$) of drug, net surface charge (Q) on drug, fraction of interstitial volume available ($K_p$) for drug, association rate and dissociation rate constants for antibody-FcRn binding at slightly acidic pH (pH = 6) ($k_{1on}$, $k_{1off}$), association rate and dissociation rate constants for antibody-antigen binding at physiological pH (pH = 7.4) ($k_{on}$, $k_{off}$) and slightly acidic pH (pH = 6) ($k_{eon}$, $k_{eoff}$), and dissociation rate constant for charge-based non-specific interactions ($K_{D,NSB}$) are considered. $k_{1on}$ and $k_{1off}$ values for antibody-FcRn binding in acidic pH and $k_{on}$, $k_{off}$, $k'_{eon}$, and $k_{eoff}$ values for antibody-antigen binding can be calculated, measured, and/or estimated accordingly and/or obtained from published literature. The size-based two-pore clearance is calculated from derived equations published previously. The quantitative relations between compound-specific parameters and PK parameters are provided in equations A49-A51.

The equations A1 to A51 of the mPBPK Model 301 are provided as follows:

*Plasma compartment*

$$\frac{dA_p}{dt} = CL_{rec} FcRnA_e/V_p - S_{pino} * k_{up,p} A_p + (L A_l - (1-\sigma_1)L_1 A_p - (1-\sigma_2)L_2 A_p)/V_p - k_{on}A_pT_p + k_{off}ATC_p \tag{A1}$$

$$\frac{dT_p}{dt} = k_{syn} - k_{on}A_pT_p + k_{off}ATC_p - (k_{p,T} - S_{pino} * k_{up,p})T_p - S_{pino} * k_{up,p}T_p \tag{A2}$$

$$\frac{dATC_p}{dt} = -k_{on}A_pT_p + k_{off}ATC_p + (L\ ATC_l - (1-\sigma_1)L_1ATC_p - (1-\sigma_2)L_2\ ATC_p)/V_p +$$

$$CL_{rec}\ FcRnATC_e/V_p - S_{pino} * k_{up,p}\ ATC_p \qquad (A3)$$

*Plasma nested endosomes*

$$\frac{dA_e}{dt} = S_{pino} * k_{up} * A_p + k_{1off}\ FcRnA_e - k_{1on}A_e\ FcRn_e - k_{deg}\ A_e - ke_{on}A_eT_e + ke_{off}ATC_e \quad (A4)$$

$$\frac{dT_e}{dt} = -ke_{on}A_eT_e + ke_{off}ATC_e - ke_{on}FcRnA_eT_e + ke_{off}FcRnATC_e - CL_{cat}T_e/V_e + S_{pino} * k_{up}T_v \quad (A5)$$

$$\frac{dATC_e}{dt} = S_{pino} * k_{up} * ATC_e + k_{1off}\ FcRnATC_e - k_{1on}ATC_e\ FcRn_e - CL_{cat}\ ATC_e\ /V_e + ke_{on}A_eT_e -$$

$$ke_{off}ATC_e \qquad (A6)$$

$$\frac{dFcRn_e}{dt} = -k_{1on}A_eFcRn_e + k_{1off}\ FcRnA_e - k_{1on}ATC_eFcRn_e + k_{1off}\ FcRnATC_e + CL_{rec} * (FcRnA_e +$$

$$FcRnATC_e)/V_e \qquad (A7)$$

$$\frac{dFcRnA_e}{dt} = k_{1on}A_eFcRn_e - k_{1off}\ FcRnA_e - ke_{on}FcRnA_eT_e + ke_{off}FcRnATC_e - CL_{rec}FcRnA_e/V_e \quad (A8)$$

$$\frac{dFcRnATC_e}{dt} = k_{1on}ATC_eFcRn_e - k_{1off}\ FcRnATC_e + ke_{on}FcRnA_eT_e - ke_{off}FcRnATC_e -$$

$$CL_{rec}FcRnATC_e/V_e \qquad (A9)$$

*Tight Tissue (vascular)*

$$\frac{dA_{v1}}{dx} = (1-\sigma_1)L_1\ A_p/(K_pVv_1) - CL_{TP1}A_{v1}/(K_pVv_1) + FR * CL_{rec}FcRnA_{e1}/(K_pVv_1) - S_{pino,1} *$$

$$k_{up}A_{v1} - k_{on}A_{v1}T_{v1} + k_{off}ATC_{v1} - kon_{nsb} * A_{v1} * (Rm_{tot}) + koff_{nsb} * ARm_{v1} - kon * A_{v1} *$$

$$Tm_{v1} + koff * ATm_{v1} \qquad (A10)$$

$$\frac{dATC_{v1}}{dx} = (1-\sigma_1)L_1\ ATC_p/(K_pVv_1) + FR * CL_{rec}FcRnATC_{e1}/(K_pVv_1) - S_{pino,1} * k_{up}ATC_{v1} +$$

$$k_{on}A_{v1}T_{v1} - k_{off}ATC_{v1} \qquad (A11)$$

$$\frac{dT_{v1}}{dt} = k_{syn} - k_{on}A_{v1}T_{v1} + k_{off}ATC_{v1} - (k_{p,T} - S_{pino,1} * k_{up})T_{v1} - S_{pino,1} * k_{up}T_{v1} \qquad (A12)$$

$$\frac{dTm_{v1}}{dt} = k_{syn.m} - k_{on}A_{v1}Tm_{v1} + k_{off}ATC_{v1} - k_{p,T}\ Tm_{v1}$$

$$(A13)$$

$$\frac{dARm_{v1}}{dt} = kon_{nsb}A_{v1}RM_{tot} - koff_{nsb}ARm_{v1} \qquad (A14)$$

$$\frac{dATm_{v1}}{dt} = k_{on}A_{v1}Tm_{v1} - k_{off}ATm_{v1} - k_{int}ATm_{v1} \tag{A15}$$

*Tight Tissue (endosomes)*

$$\frac{dA_{e1}}{dt} = S_{pino} * k_{up} * A_{v1} + k_{1off}FcRnA_{e1} - k_{1on}A_{e1}FcRn_{e1} - k_{deg}A_{e1} - ke_{on}A_{e1}T_{e1} +$$

$$ke_{off}ATC_{e1} \tag{A16}$$

$$\frac{dT_{e1}}{dt} = -ke_{on}A_{e1}T_{v1} + ke_{off}ATC_{e1} - ke_{on}FcRnA_{e1}T_{e1} + ke_{off}FcRnATC_{e1} - CL_{cat}T_{e1}/V_{e1} + S_{pino,1} *$$

$$k_{up}T_{v1} \tag{A17}$$

$$\frac{dATC_{e1}}{dt} = S_{pino,1} * k_{up} * ATC_{v1} + k_{1off}FcRnATC_{e1} - k_{1on}ATC_{e1}FcRn_{e1} - CL_{cat}ATC_{e1}/V_{e1} +$$

$$ke_{on}A_{e1}T_{e1} - ke_{off}ATC_{e1} \tag{A18}$$

$$\frac{dFcRn_{e1}}{dt} = -k_{1on}A_{e1}FcRn_{e1} + k_{1off}FcRnA_{e1} - k_{1on}ATC_{e1}FcRn_{e1} + k_{1off}FcRnATC_{e1} + CL_{rec} *$$

$$(FcRnA_{e1} + FcRnATC_{e1})/V_{e1} \tag{A19}$$

$$\frac{dFcRnA_{e1}}{dt} = k_{1on}A_{e1}FcRn_{e1} - k_{1off}FcRnA_{e1} - ke_{on}FcRnA_{e1}T_{e1} + ke_{off}FcRnATC_{e1} -$$

$$CL_{rec}FcRnA_{e1}/V_{e1} \tag{A20}$$

$$\frac{dFcRnATC_{e1}}{dt} = k_{1on}ATC_{e1}FcRn_{e1} - k_{1off}FcRnATC_{e1} + ke_{on}FcRnA_{e1}T_{e1} - ke_{off}FcRnATC_{e1} -$$

$$CL_{rec}FcRnATC_{e1}/V_{e1} \tag{A21}$$

*Tight tissue (interstitium)*

$$\frac{dA_{is1}}{dt} = -\frac{(1-\sigma_L)L_1A_{is1}}{(K_pV_{is1})} - S_{pino,1} * k_{up}A_{is1} + \frac{(1-FR)CL_{rec}FcRnA_{e1}}{K_pV_{is1}} + CL_{TP1}A_{is1}/(K_pV_{is1}) \tag{A22}$$

$$\frac{dATC_{is1}}{dt} = -(1-\sigma_L)L_1ATC_{is1}/(K_pV_{is1}) - S_{pino,1} * k_{up}ATC_{is1} + (1-FR)CL_{rec}FcRnATC_{e1}/$$

$$(K_pV_{is1}) \tag{A23}$$

*Leaky Tissue (vascular)*

$$\frac{dA_{v2}}{dx} = (1-\sigma_2)L_2 A_p/(K_pVv_2) - (CL_{renal} + CL_{TP2})A_{v2}/(K_pVv_2) + FR * CL_{rec}FcRnA_{e2}/$$

$$(K_pVv_2) - S_{pino,2} * k_{up}A_{v2} - k_{on}A_{v2}T_{v2} + k_{off}ATC_{v2} - kon_{nsb} * A_{v2} * (Rm_{tot}) + koff_{nsb} *$$

$$ARm_{v2} - kon * A_{v2} * Tm_{v2} + koff * ATm_{v2} \tag{A24}$$

$$\frac{dATC_{v2}}{dx} = (1-\sigma_2)L_2 ATC_p/(K_pVv_2) + FR * CL_{rec}FcRnATC_{e2}/(K_pVv_2) - S_{pino,2} * k_{up}ATC_{v2} +$$

$$k_{on}A_{v2}T_{v2} - k_{off}ATC_{v2} \tag{A25}$$

$$\frac{dT_{v2}}{dt} = k_{syn} - k_{on}A_{v2}T_{v2} + k_{off}ATC_{v2} - (k_{p,T} - S_{pino,2} * k_{up})T_{v2} - S_{pino,2} * k_{up}T_{v2} \qquad (A26)$$

$$\frac{dTm_{v2}}{dt} = k_{syn.m} - k_{on}A_{v2}Tm_{v2} + k_{off}ATC_{v2} - k_{p,T} Tm_{v2} \qquad (A27)$$

$$\frac{dARm_{v2}}{dt} = kon_{nsb}A_{v2}RM_{tot} - koff_{nsb}ARm_{v2} \qquad (A28)$$

$$\frac{dATm_{v2}}{dt} = k_{on}A_{v2}Tm_{v2} - k_{off}ATm_{v2} - k_{int}ATm_{v2} \qquad (A29)$$

*Leaky Tissue (endosomes)*

$$\frac{dA_{e2}}{dt} = S_{pino,2} * k_{up} * A_{v2} + k_{1off} FcRnA_{e2} - k_{1on}A_{e2} FcRn_{e2} - k_{deg} A_{e2} - ke_{on}A_{e2}T_{e2} + ke_{off}ATC_{e2} \qquad (A30)$$

$$\frac{dT_{e2}}{dt} = -ke_{on}A_{e2}T_{v2} + ke_{off}ATC_{e2} - ke_{on}FcRnA_{e2}T_{e2} + ke_{off}FcRnATC_{e2} - \frac{CL_{cat}T_{e2}}{V_{e2}} + S_{pino,2} * k_{up}T_{v2} \qquad (A31)$$

$$\frac{dATC_{e2}}{dt} = S_{pino,2} * k_{up} * ATC_{v2} + k_{1off} FcRnATC_{e2} - k_{1on}ATC_{e2} FcRn_{e2} - CL_{cat} ATC_{e2}/V_{e2} + ke_{on}A_{e2}T_{e2} - ke_{off}ATC_{e2} \qquad (A32)$$

$$\frac{dFcRn_{e2}}{dt} = -k_{1on}A_{e2}FcRn_{e2} + k_{1off} FcRnA_{e2} - k_{1on}ATC_{e2}FcRn_{e2} + k_{1off} FcRnATC_{e2} + CL_{rec} * (FcRnA_{e2} + FcRnATC_{e2})/V_{e2} \qquad (A33)$$

$$\frac{dFcRnA_{e2}}{dt} = k_{1on}A_{e2}FcRn_{e2} - k_{1off} FcRnA_{e2} - ke_{on}FcRnA_{e2}T_{e2} ke_{off}FcRnATC_{e2} - CL_{rec}FcRnA_{e2}/V_{e2} \qquad (A34)$$

$$\frac{dFcRnATC_{e2}}{dt} = k_{1on}ATC_{e2}FcRn_{e2} - k_{1off} FcRnATC_{e2} + ke_{on}FcRnA_{e2}T_{e2} - ke_{off}FcRnATC_{e2} - CL_{rec}FcRnATC_{e2}/V_{e2} \qquad (A35)$$

*Leaky tissue (interstitium)*

$$\frac{dA_{is2}}{dt} = -(1 - \sigma_L)L_2A_{is2}/(K_pV_{is2}) - S_{pino,2} * k_{up}A_{is2} + (1 - FR)CL_{rec}FcRnA_{e2}/(K_pV_{is2}) + CL_{TP2}A_{is2}/(K_pV_{is2}) \qquad (A36)$$

$$\frac{dATC_{is2}}{dt} = -(1 - \sigma_L)L_2ATC_{is2}/(K_pV_{is2}) - S_{pino,2} * k_{up}ATC_{is2} + (1 - FR)CL_{rec}FcRnATC_{e2}/$$

$$(K_pV_{is2}) \tag{A37}$$

*Lymph compartment*

$$\frac{dA_l}{dt} = (1 - \sigma_L) * L1 * \frac{A_{is1}}{V_L} + (1 - \sigma_L) * L2 * \frac{A_{is2}}{V_L} - L * \frac{A_l}{V_L} \tag{A38}$$

$$\frac{dATC_l}{dt} = (1 - \sigma_L) * L1 * \frac{ATC_{is1}}{V_L} + (1 - \sigma_L) * L2 * \frac{ATC_{is2}}{V_L} - L * \frac{ATC_l}{V_L} \tag{A39}$$

In the above differential equations A1 to A39 describing the mPBPK model 301, A, T, ATC, FcRnA, and FcRnATC are free antibody (A), free soluble antigen target (T), antibody-target complex (ATC), FcRn-antibody complex (FcRnA), and FcRn-antibody-target complex (FcRnATC), respectively. $C_{e1}$, $C_{e2}$ are endosomal concentrations, $C_{v1}$, $C_{v2}$ are vascular concentrations, $C_{is1}$, $C_{is2}$ are interstitial concentrations in tight and leaky tissue compartments, respectively. $CL_{renal}$ is the renal clearance rate, $CL_{rec}$ is the endosomal recycling / clearance rate, $CL_{cat}$ is the catabolism rate of antibody-antigen complex, and $CL_{TP}$ is the two-pore clearance (equation A47-A48). The effect of charge on antibody PK is reflected in the mPBPK model 301 through a modulation factor ($K_p$) for interstitial volume of distribution, a modulation factor ($S_{pino}$) for pinocytosis rate, and a charge-dependent non-specific equilibrium dissociation constant ($K_{D,NSB}$). The subscripts p, 1, 2, L, v, is, and e stand for plasma, tight tissues, leaky tissues, lymph, vascular, interstitial, and endosomal compartment, respectively.

*Calculation for two-pore mediated clearance*

$$J_{l,1} = J_{iso,1} + (\alpha_l) * L1 \tag{A40}$$

$$J_{s,1} = J_{iso,1} + (1 - \alpha_l) * L1 \tag{A41}$$

$$J_{l,2} = J_{iso,2} + (\alpha_l) * L2 \tag{A42}$$

$$J_{s,2} = J_{iso,2} + (1 - \alpha_l) * L2 \tag{A43}$$

$$a_e = 0.0483 * MW^{0.386} \tag{A44}$$

$$\sigma_l = 0.000035 * MW^{0.717} \tag{A45}$$

$$\sigma_s = 1 - 0.8489 * e^{-0.00004*MW} \tag{A46}$$

$J_{l,i}$, $J_{s,i}$, are lymph flow rate through large and small pores, respectively. $a_e$ is the Stoke's radius of a protein. $\sigma_l$, $\sigma_s$ are vascular reflection coefficient of large and small pores, respectively. Molecular weight (MW) is in g/mol or Dalton units. In the above, i = 1 represents tight tissues and i =2 represents leaky tissues.

Two-pore-clearance rate ($CL_{TP}$) via large and small pores

$$CL_{TP_L,i} = PS_{L,i}\left(1 - \frac{C_{is,i}}{C_{v,i}}\right)\frac{Pe_L}{e^{Pe_L}-1} + J_{L,i}(1 - \sigma_L) \tag{A47}$$

$$CL_{TP_{S,i}} = PS_{S,i}\left(1 - \frac{C_{is,i}}{C_{v,i}}\right)\frac{Pe_S}{e^{Pe_S}-1} + J_{S,i}(1 - \sigma_S) \tag{A48}$$

$PS_{L,i}$, $PS_{S,i}$, $Pe_L$, $Pe_S$, $\sigma_L$, $\sigma_S$ may be calculated or estimated accordingly or found in literature studies. In this example, $PS_{L,i}$, $PS_{S,i}$, $Pe_L$, $Pe_S$, $\sigma_L$, $\sigma_S$ were calculated using derived equations from a literature study by Z. Li and D. K. Shah, "Two-pore physiologically based pharmacokinetic model with de novo derived parameters for predicting plasma PK of different size protein therapeutics.," Journal of pharmacokinetics and pharmacodynamics, pp. 305-218, 2019. In this case, i = 1 represents tight tissues and i =2 for represents leaky tissues. In this example, the two-pore transport model related parameters for the species of interest (e.g., mice) used in eq. A40 - A48 are provided as:

| Parameters | Definition | Value |
|---|---|---|
| $r_l$ | Large pore radius | 22.85 nm |
| $r_s$ | Small pore radius | 4.44 nm |
| $D$ | Diffusion coefficient | 1.92e-5 cm2/min |
| $\eta$ | Viscosity | 1.12e-9 N*min/cm2 |
| $\alpha_l$ | Fraction of hydraulic conductivity | 0.042 |
| $J_{iso, tight}$ | recirculation flow rate (L/h) | 0.38*L1 |
| $J_{iso,leaky}$ | recirculation flow rate (L/h) | 0.38*L2 |

The above example values for the corresponding physiology-based and kinetic parameters may be calculated, measured, estimated and/or found from relevant literature studies in relation to the subject of the species of interest (e.g., in this example, the subject of the species of interest is mice) for input to the mPBPK model 301.

Quantitative equations to relate antibody properties (e.g., size (ae), charge (Q)) to PK parameters:

$$\Theta = 0.926 * e^{-\left(\frac{a_e - 1.25}{1.25}\right)^2} \tag{A49}$$

where $\Theta$ is the sieving coefficient of the renal filtration membrane

$$1/K_{D,NSB} = \frac{1}{0.059*e^{0.51*Q}} + 0.06606 \tag{A50}$$

$$K_p = -0.0067 * Q^2 - 0.0063 * Q + 1 \tag{A51}$$

In this example, the mPBPK model 301 is configured to used equations A1 to A51 to simulate and capture the effect of antibody size on plasma and tissue PK. The mPBPK model 301 is configured to predict the PK in plasma and tissues for large molecules such as, for exampe, a full-length IgG (150 kDa) and a one-armed IgG (100 kDa). For larger sized antibodies (> 100 kDa), size-based renal clearance is insignificant. The larger size of these antibodies restricts their movement through size-selective glomerular filtration membrane in the kidneys and other small pores. The mPBPK model 301 can demonstrate the plasma and tissue PK of smaller-sized IgG fragments (50 kDa) without an intact Fc region. Additionally, the mPBPK model 301 has validated that the PK of FcRn non-binding antibody fragments with a size ranging between 50-100 kDa. The mPBPK model 301 is configured to quantitatively relate the effect of charge (Q) on non-specific binding affinity (eq. A50), and fraction of volume of distribution (eq. A51) through empirical equations.

As can be seen, the mPBPK model 301 takes in the physiology-based parameters (e.g., organ volumes, tissue lymph flows, blood flows), antibody-specific properties (e.g., MW, size, charge, binding affinity), and target properties (e.g., baseline expression, half-life) and based on equations A1-A51 the mPBPK model simulates and predicts the antibody PK profile, PK endpoints and target occupancy as a percentage (e.g., TO%). Various PK values calculated include, without limitation, for example area under the curve at steady state ($AUC_{ss}$), minimum plasma concentration ($C_{min}$), maximum plasma concentration ($C_{max}$), time to reach minimum plasma concentration ($t_{min}$), total soluble ($T_{k,supp}$) and membrane target suppressed ($Tm_{k,supp}$). The mPBPK model 301 is configured to compute various PK endpoints and/or pharmacological/pharmacodynamic effects and/or values. For example, using the below equations A53-A57, the mPBPK model 301 may output a set of PK parameters/values including, without limitation, for example at least $C_{min}$, $AUC_{ss}$, $C_{max}$, $C_{ss}$, $T_{k,supp}$, and $Tm_{k,supp}$. As well, the mPBPK model 301 may compute the pharmacological/pharmacodynamic effects including, without limitation, for example target occupancy, TO%, using equation A52, which is based on the ratio of bound target and total target in plasma, tight tissues, and leaky tissues, where TO% is calculated at minimum and maximum concentration of drug in plasma for each scenario. In this example, the mPBPK model 301 computes using equations A52 to A57 estimates for target occupancy as a percentage (TO%) and $AUC_{ss}$, $C_{min}$, $C_{max}$, $T_{k,supp}$, and $Tm_{k,supp}$ based on:

$$TO_k\,(\%) = \frac{ATC_k(t)}{ATC_k(t)+T_k(t)} \qquad\qquad A52$$

$$AUC_{ss} = \int_0^\infty A_p\,dt \qquad\qquad A53$$

$$C_{min} = \min\left(A_p(t)\right) \qquad\qquad A54$$

$$C_{max} = \max\left(A_p(t)\right) \qquad\qquad A55$$

$$T_{k,supp} = \max(T_k(t)) - \min\,(T_k(t)) \qquad\qquad A56$$

$$Tm_{k,supp} = \max(Tm_k(t)) - \min\,(Tm_k(t)) \qquad\qquad A57$$

where $TO_k\,(\%)$ is the target occupancy (%) calculated at time (t) and site (k) such as plasma, tight tissue, leaky tissue, *ATC* is concentration of drug-target complex and T is the concentration of target. $AUC_{ss}$ is the AUC exposure of antibody in plasma calculated at steady state. Target suppression for soluble (T) and membrane-bound (Tm) target is computed as difference between maximum and minimum of total target concentration at site (k) such as plasma, tight tissue, and leaky tissue. $TO_k(\%)$, $AUC_{ss}$, $C_{min}$, $C_{max}$, $T_{k,supp}$, and $Tm_{k,supp}$ are computed within the mPBPK model 301 based on, for example, equations A1-A57.

Although the pharmacological effect of target occupancy (TO%) and the PK values/endpoints $AUC_{ss}$, $C_{min}$, $C_{max}$, $T_{k,supp}$, and $Tm_{k,supp}$ are described as being computed by the mPBPK model 301, this is by way of example only and the mPBPK model 301 is not so limited, it is to be appreciated by the skilled person that the mPBPK model 301 may be configured to compute any suitable pharmacological/pharmacodynamic effect/value (e.g., TO%, toxicity or efficacy values/endpoints) and/or any suitable PK profiles, endpoints, parameters and/or properties associated with ADME/-LADME and the like, as described herein, combinations thereof, modifications thereto, and/or as the application demands: Although the mPBPK model is described herein, this is by way of example only and the invention is not so limited, it is to be appreciated by the skilled person that the PBPK/ML model systems 200 and 230 and/or processes 100, 105, 110, 120 as described with reference to figures 1a to 2e may use any other suitable PBPK model for simulating the interaction of molecule drugs and targets in a physiological or cellular system of a subject of a species of interest and computing the requisite sets of PK values of corresponding PK profiles/endpoints and corresponding pharmacological/pharmacodynamic values such as, for example, target engagement response/target occupancy and/or as the application demands.

Although the PBPK model and ML models described herein and processes 100, 105, 110, 120, PBPK and ML systems 200 and 250, and PBPK model 301 are described with reference to antibodies, in particular, large monoclonal antibodies and the like, this is by way of example only and the invention is not so limited, it is to be appreciated by the skilled person that the PBPK model and ML model and processes 100, 105, 110, 120, PBPK and ML systems 200 and 250, and PBPK model 301 as described herein may be applicable to any molecule that may be described in terms of at least a set of physiochemical properties and a set of PK values associated with a drug dosage regimen and target such as, without limitation, for example antibodies, CODV antibodies, enzymes, other proteins, and/or any other suitable protein format, combinations thereof, modifications thereto, and/or the like and/or as the application demands.

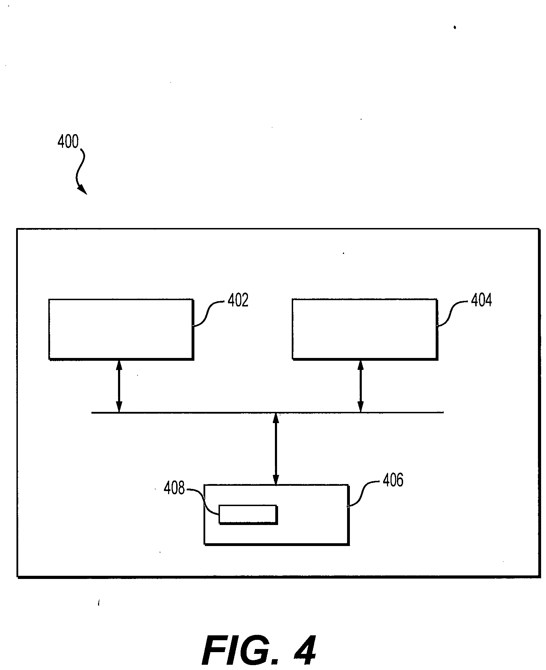

Figure 4 is a schematic illustration of a system/apparatus for performing methods described herein. The system/apparatus shown is an example of a computing device. It will be appreciated by the skilled person that other types of computing devices/systems may alternatively be used to implement the methods described herein, such as a distributed computing system.

The apparatus (or system) 400 comprises one or more processors 402. The one or more processors control operation of other components of the system/apparatus 400. The one or more processors 402 may, for example, comprise a general-purpose processor. The one or more processors 402 may be a single core device or a multiple core device. The one or more processors 402 may comprise a central processing unit (CPU) or a graphical processing unit (GPU).

Alternatively, the one or more processors 402 may comprise specialised processing hardware, for instance a RISC processor or programmable hardware with embedded firmware. Multiple processors may be included.

The system/apparatus comprises a working or volatile memory 404. The one or more processors may access the volatile memory 404 in order to process data and may control the storage of data in memory. The volatile memory 404 may comprise RAM of any type, for example Static RAM (SRAM), Dynamic RAM (DRAM), or it may comprise Flash memory, such as an SD-Card.

The system/apparatus comprises a non-volatile memory 406. The non-volatile memory 406 stores a set of operation instructions 408 for controlling the operation of the processors 402 in the form of computer readable instructions. The non-volatile memory 406 may be a memory of any kind such as a Read Only Memory (ROM), a Flash memory or a magnetic drive memory.

The one or more processors 402 are configured to execute operating instructions 608 to cause the system/-apparatus to perform any of the methods or processes described herein with reference to figures 1a to 3. The operating instructions 408 may comprise code (i.e., drivers) relating to the hardware components of the system/apparatus 400, as well as code relating to the basic operation of the system/apparatus 400. Generally speaking, the one or more processors 402 execute one or more instructions of the operating instructions 408, which are stored permanently or semi-permanently in the non-volatile memory 406, using the volatile memory 404 to temporarily store data generated during execution of said operating instructions 408.

Implementations of the methods described herein may be realised as in digital electronic circuitry, integrated circuitry, specially designed ASICs (application specific integrated circuits), computer hardware, firmware, software, and/or combinations thereof. These may include computer program products (such as software stored on e.g., magnetic discs, optical disks, memory, Programmable Logic Devices) comprising computer readable instructions that, when executed by a computer, such as that described in relation to Figure 4, cause the computer to perform one or more of the methods described herein.

Any system feature as described herein may also be provided as a method feature, and vice versa. As used herein, means plus function features may be expressed alternatively in terms of their corresponding structure. In particular, method aspects may be applied to system aspects, and vice versa.

Furthermore, any, some and/or all features in one aspect can be applied to any, some and/or all features in any other aspect, in any appropriate combination. It should also be appreciated that particular combinations of the various features described and defined in any aspects of the invention can be implemented and/or supplied and/or used independently.

Although several embodiments have been shown and described, it would be appreciated by those skilled in the art that changes may be made in these embodiments without departing from the principles of this disclosure, the scope of which is defined in the claims.

## Claims

1. A computer-implemented method of classifying one or more candidate molecule drugs in relation to one or more desired conditions associated with pharmacological or pharmacodynamic effects for drug optimization, the method comprising:

   receiving a candidate molecule drug inference dataset representing one or more candidate molecule drugs, wherein each candidate molecule drug in the candidate molecule drug dataset is represented by a set of physiochemical values for said candidate molecule drug and a set of pharmacokinetic, PK, values with a dosage regime and target in relation to said each candidate molecule drug and wherein each candidate molecule drug of the candidate molecule drug dataset is classified as a large molecule drug;
   applying, for each candidate molecule drug of the candidate molecule drug inference dataset, the corresponding set of physiochemical values and set of PK values for the dosage regimen and target to a machine learning, ML, model configured for classifying whether said each candidate molecule drug meets one or more desired conditions associated with pharmacological or pharmacodynamic effects, wherein the ML model is trained using an ML algorithm to train model parameters defining the ML model for classifying whether each candidate molecule drug meets the one or more desired conditions associated with pharmacological or pharmacodynamic effects based on a training dataset comprising data representative of a plurality of molecule drugs represented by known and/or computed sets of physiochemical values and sets of PK values associated with the dosage regimen and annotated based on the one or more desired conditions associated with pharmacological or pharmacodynamic effects; and
   outputting data representative of a set of candidate molecule drugs classified according to the desired conditions associated with pharmacological or pharmacodynamic effects for downstream processing for drug optimization.

2. The computer-implemented method of claim 1, wherein each candidate molecule drug is an antibody.

3. The computer-implemented method of claims 1 or 2, wherein each candidate molecule drug is a monoclonal antibody.

4. The computer-implemented method of claim 3, wherein the monoclonal antibody has a size greater than or equal to 50KDa.

5. The computer-implemented method of claims 3 or 4, wherein the monoclonal antibody is classified as a large molecule drug.

6. The computer-implemented method of any preceding claim, wherein the target is an antigen.

7. The computer-implemented method of any of claims 1 to 6, wherein the set of physiochemical values for each molecule drug correspond with one or more physiochemical parameters or properties from the group of:

   molecular weight, MW;
   size or Stokes radius, ae;
   charge, Q;
   hydrophobicity;
   binding affinities;
   isoelectric point, pl;
   glycosylation;
   drug-target binding constant, KD; and
   any other type of physiochemical parameter or property associated with each molecule drug.

8. The computer-implemented method of claim 7, wherein the set of physiochemical properties for each molecule drug comprises at least MW, ae, Q, and KD.

9. The computer-implemented method of any of claims 1 to 8, wherein the set of PK values of each molecule drug are associated with PK profiles, endpoints, parameters and/or properties corresponding with absorption, distribution, metabolism, and excretion, ADME, or liberation, absorption, distribution, metabolism, and excretion, LADME.

10. The computer-implemented method of any of claims 1 to 6, wherein the PK profiles, endpoints, parameters and/or properties include one or more from the group of:

    dosage, D;
    dosing interval, $\tau$;
    maximum serum concentration, $C_{max}$;
    minimum time for $C_{max}$;
    minimum plasma concentration, $C_{min,ss}$;
    average plasma concentration, $C_{av,ss}$;
    concentration at steady state, $C_{ss}$;
    volume of distribution, $V_d$;
    time to reach minimum plasma concentration, $t_{min}$;
    total soluble, $T_{k,supp}$;
    membrane target suppressed, $Tm_{k,supp}$;
    absorption half-life;
    absorption rate constant;
    elimination half-life;
    elimination rate constant;
    area under the concentration time curve after a single dose, or in steady state, $AUC_{ss}$;
    Clearance, C; and
    any other type of PK profile, endpoint, parameter and/or property associated with ADME or LADME.

11. The computer-implemented method of claim 10, wherein the set of PK profiles, endpoints, parameters and/or properties for each molecule drug comprises at least $C_{min}$, $AUC_{ss}$, $C_{max}$, $C_{ss}$, $T_{k,supp}$, and $Tm_{k,supp}$.

12. The computer-implemented method of any preceding claim, wherein the ML algorithm comprising one or more ML algorithms or combinations thereof from the group of:

> neural network based ML algorithm;
> decision tree-based classification ML algorithm;
> random forest decision tree ML algorithm;
> Adaptive Boosting based ML algorithm;
> Gradient Boosting based ML algorithm;
> Bagging based ML algorithm;
> Ensemble based ML algorithm;
> any other supervised ML algorithm suitable for training model parameters of an ML model for outputting a classification whether one or more molecule drugs meet one or more desired conditions associated with pharmacological or pharmacodynamic effects when data representative of a set of physiochemical values and set of PK values for the target and dosage regime corresponding to the one or more molecule drugs are input.

13. The computer-implemented method of any preceding claim, further comprising iteratively training model parameters defining the ML model using the ML algorithm associated with the ML model, the training comprising:

> receiving a training dataset associated with a plurality of molecule drugs, the training dataset comprising a plurality of training instances, each training instance associated with each molecule drug and comprising data representative of a known and/or desired set of physiochemical values and a corresponding known or computed set of PK values associated with the dosage regimen and the target, and annotated or labelled based on whether each molecule drug with target meets desired conditions associated with pharmacological or pharmacodynamic effects;
> iteratively applying the training dataset to the ML algorithm for training the model parameters of the ML model until a stopping criterion is reached; and
> outputting the model parameters representative of the trained ML model.

14. The computer-implemented method as claimed in any preceding claim, wherein the sets of physiochemical values corresponding to the molecule drugs of the training dataset are based on synthetically generated molecule drugs generated over a desired range physiochemical values, wherein, for each synthetically generated molecule drug, the corresponding PK values associated with the dosage regimen and the target, and the associated with pharmacological or pharmacodynamic effects, responses and/or values corresponding to said each synthetically generated molecule drug are computed using a physiologically based pharmacokinetic, PBPK, model configured for simulating the effects of said synthetically generated molecule drug on the target in a cell system of a subject and outputting, for said each synthetically generated molecule, a corresponding set of PK values from selected PK endpoints and profiles and a corresponding pharmacological or pharmacodynamic effects, responses and/or values associated with the desired conditions.

15. The computer-implemented method as claimed in any preceding claim, wherein the sets of physiochemical values, PK values associated with the dosage regimen and the target, and associated with pharmacological or pharmacodynamic effects, responses and/or values corresponding to each molecule drug of the training dataset are based on known or measured physiochemical values, PK values associated with the dosage regimen and the target, and associated with pharmacological or pharmacodynamic effects, responses and/or values of real-world molecule drugs.

16. The computer-implemented method as claimed in any preceding claim, wherein the sets of physiochemical values corresponding to the molecule drugs of the training dataset are based on real-world molecule drugs meeting a desired range of physiochemical values, wherein, for each real-world molecule drug, the corresponding PK values associated with the target and dosage regimen and the pharmacological or pharmacodynamic effects, responses and/or values corresponding to said each real-world molecule drug are computed using a PBPK model configured for simulating the effects of said real-world molecule drug on the target in a physiological or cellular system of a subject and outputting, for said each real-world molecule drug, a corresponding set of PK values from selected PK endpoints and profiles and a corresponding pharmacological or pharmacodynamic effects, responses and/or values.

17. The computer-implemented method as claimed in any preceding claim, wherein the sets of physiochemical values corresponding to the molecule drugs of the candidate molecule drug inference dataset are based on synthetically generated molecule drugs generated over a desired range physiochemical values, wherein, for each synthetically generated molecule drug, the corresponding PK values associated with the dosage regimen and the corresponding pharmacological or pharmacodynamic effects, responses and/or values corresponding to said each synthetically generated molecule drug are computed using a PBPK model configured for simulating the effects of said synthetically generated molecule drug on the target in a physiological or cellular system of a subject and outputting, for said each synthetically generated molecule, a corresponding set of PK values from selected PK endpoints and profiles and corresponding pharmacological or pharmacodynamic effects, responses and/or values.

18. The computer-implemented method as claimed in any preceding claim, wherein the sets of physiochemical values, PK values associated with the dosage regimen and the target, and the pharmacological or pharmacodynamic effects, responses and/or values corresponding to each molecule drug of the candidate molecule drug inference dataset are based on known or measured physiochemical values, PK values associated with the dosage regimen and the target, and pharmacological or pharmacodynamic effects, responses and/or values of real-world molecule drugs.

19. The computer-implemented method as claimed in any preceding claim, wherein the sets of physiochemical values corresponding to the molecule drugs of the candidate molecule drug inference dataset are based on real-world molecule drugs meeting a desired range physiochemical values from a set of physiochemical parameters, wherein, for each real-world molecule drug, the corresponding PK values associated with the target and dosage regimen and the pharmacological or pharmacodynamic effects, responses and/or values corresponding to said each real-world molecule drug are computed using the PBPK model configured for simulating the effects of said real-world molecule drug on the target in a cell system of a subject and outputting, for said each real-world molecule drug, a corresponding set of PK values from selected PK endpoints and profiles and corresponding pharmacological or pharmacodynamic effects, responses and/or values.

20. The computer-implemented method according to any preceding claim, further comprising:

    receiving a set of physiochemical values corresponding to each molecule drug;
    receiving one or more sets of physiochemical values corresponding to the target;
    receiving a set of dosage regimens;
    generating, for each desired dosage regimen, one or more unique pairs of molecule drug and the target data based on pairing the set of desired physiochemical values of the molecule drug with each one of the one or more sets desired physiochemical values of the target;
    applying, for each dosage regimen, the set of desired physiochemical values of the molecule drug and the set of desired physiochemical values of the target for each pair of molecule drug and target data to the PBPK model;
    computing, for each dosage regimen and said each pair of molecule drug and target data, a set of PK values and the corresponding pharmacological or pharmacodynamic effects, responses and/or values associated with the desired conditions for said each pair of molecule drug and target data using the PBPK model;
    generating, for each pair of molecule drug and target and corresponding dosage regimen, either a training data instance comprising data representative of at least the set of physiochemical properties for each molecule drug, the computed set of PK values and annotated based on the corresponding pharmacological or pharmacodynamic effects, responses and/or values and the desired conditions computed for each pair of molecule drug and target and corresponding dosage regimen, or a candidate molecule drug inference data instance comprising data representative of at least the set of physiochemical properties for each molecule drug and the computed set of PK values computed for each pair of molecule drug and target and corresponding dosage regimen;
    storing the training data instances or candidate molecule drug inference data instances for use in training or inference/classification, respectively.

21. The computer-implemented method of any preceding claim, wherein outputting the set of candidate molecule drugs satisfying the desired conditions associated with pharmacological or pharmacodynamic effects for downstream drug optimization further comprises outputting a shortlist of candidate molecule drugs, each candidate molecule drug represented by a corresponding set of physiochemical values and set of PK values for the dosage regimen and target for use in downstream processing including *in vitro* wet lab analysis or high-throughput screening.

22. The computer-implemented method of any of claims 1 to 20, wherein outputting the set of candidate molecule drugs satisfying the desired conditions associated with pharmacological or pharmacodynamic effects for downstream drug optimization further comprises outputting a shortlist of candidate molecule drugs, each candidate molecule drug represented by a corresponding set of physiochemical values and a set of PK values for the dosage regimen and target for use in downstream processing including *in vivo* trials.

23. The computer-implemented method of claims 21 or 22, wherein when one or more of the shortlist of candidate molecule drugs are represented by only a set of physiochemical values and a set of PK values, then prior to either *in vitro* wet lab analysis, or high-throughput screening, or *in vivo* trials, generating one or more real-world molecule drugs meeting the corresponding set of physiochemical values, set of PK values of the one or more candidate molecule drugs.

24. The computer-implemented method of claim 23, wherein generating one or more real-world molecule drugs further comprising computing, for each corresponding candidate molecule drug, the structural and chemical properties required to realise or synthesise a real-world molecule drug meeting the corresponding set of physiochemical values, set of PK values and associated pharmacological or pharmacodynamic effects, responses and/or values.

25. The computer-implemented method of any of claims 1 to 20, wherein outputting the set of candidate molecule drugs satisfying the desired conditions for downstream drug optimization further comprises outputting data representing either:

a shortlist of candidate molecule drugs with PK values for the dosage regimen and target, and associated pharmacological or pharmacodynamic effects, responses and/or values meeting the desired conditions for review by one or more users or expert systems further assessing the output shortlist of candidate molecule drugs; or

a list of candidate molecule drugs with PK values for the dosage regimen and target, and associated pharmacological or pharmacodynamic effects, responses and/or values classified as to whether or not they meet the desired conditions for review by one or more users or expert systems further assessing the output list of candidate molecule drugs.

26. An apparatus comprising a processor, a memory unit and a communication interface,
wherein the processor is connected to the memory unit and the communication interface, wherein the processor and memory are configured to implement the computer-implemented method according to any of the preceding claims.

27. A computer-readable medium comprising data or instruction code, which when executed on a processor, causes the processor to implement the computer-implemented method of any of claims 1 to **25Error! Reference source not found..**

28. A computing system comprising:

a molecule and target generation unit configured for generating one or more molecules represented by corresponding sets of physiochemical values and one or more targets represented by desired physiochemical values;

a physiologically based pharmacokinetic, PK, unit configured for generating a set of PK values and/or associated pharmacological or pharmacodynamic effects, responses, and/or values for each pair of molecule drug and targets as either training data instances or candidate molecule drug inference data instances;

a ML module configured for either training an ML model for classifying whether one or more molecule drugs meets desired conditions associated with pharmacological or pharmacodynamic effects for a particular dosage regimen using a training dataset of a plurality of training data instances, or inferring using a trained ML model to classify, for a particular dosage regimen, whether one or more input candidate molecule drug inference data instances meets the desired target conditions; and

an output module configured to output at least a shortlist of molecule drugs meeting the desired conditions associated with pharmacological or pharmacodynamic effects, each molecule drug represented by a set of physiochemical values and a set of PK values for use in generating real-world molecules with corresponding characteristics for *in vivo* trials, *in vitro* wet lab analysis, and/or high-throughput screening laboratory analysis.

29. A non-transitory tangible computer-readable medium comprising data or instruction code for classifying one or more candidate molecule drugs in relation to one or more desired conditions associated with pharmacological or pharmacodynamic effects for drug optimization, which when executed on one or more processors, causes at least one of the processors to perform at least the operations of:

receiving a candidate molecule drug inference dataset representing one or more candidate molecule drugs, wherein each candidate molecule drug in the candidate molecule drug dataset is represented by a set of physiochemical values for said candidate molecule drug and a set of pharmacokinetic, PK, values with a dosage regimen and target in relation to said each candidate molecule drug;

applying, for each candidate molecule drug of the candidate molecule drug inference dataset, the corresponding set of physiochemical values and set of PK values for the dosage regimen and target to a machine learning, ML, model configured for classifying whether said each candidate molecule drug meets one or more desired conditions associated with pharmacological or pharmacodynamic effects, wherein the ML model is trained using an ML algorithm to train model parameters defining the ML model for classifying whether each candidate molecule drug meets the one or more desired conditions based on a training dataset comprising data representative of a plurality of molecule drugs represented by known and/or computed sets of physiochemical values and sets of PK values associated with the dosage regimen and the target and annotated in relation to corresponding pharmacological or pharmacodynamic effects, responses and/or values in relation to the desired conditions; and

outputting data representative of a set of candidate molecule drugs satisfying the desired conditions for downstream processing for drug optimization.

100

101

RECEIVING A CANDIDATE MOLECULE DRUG DATASET INCLUDING, FOR EACH CANDIDATE MOLECULE DRUG, CORRESPONDING PHYSIOCHEMICAL VALUES AND PHARMACOKINETIC (PK) VALUES

102

INPUTTING, FOR EACH CANDIDATE MOLECULE DRUG IN THE DATASET, THE CORRESPONDING PHYSIOCHEMICAL VALUES AND PK VALUES TO AN ML MODEL FOR CLASSIFYING WHETHER SAID EACH CANDIDATE MOLECULE DRUG SATISFIES ONE OR MORE DESIRED CONDITIONS RELATED TO PHARMACOLOGICAL EFFECTS

103

OUTPUTTING A SET OF CANDIDATE MOLECULE DRUGS SATISFYING THE DESIRED CONDITIONS FOR DOWNSTREAM PROCESSING FOR DRUG OPTIMISATION

# FIG. 1A

105

106 — RECEIVING A TRAINING DATASET INCLUDING DATA REPRESENTATIVE OF PHYSIOCHEMICAL VALUES, PK VALUES ASSOCIATED WITH A DOSAGE REGIMEN AND TARGET, AND ANNOTATED ACCORDING TO ONE OR MORE CONDITIONS ASSOCIATED WITH PHARMACOLOGICAL/PHARMACODYNAMIC EFFECTS FOR A PLURALITY OF MOLECULE DRUGS

107 — ITERATIVELY APPLYING THE TRAINING DATASET TO AN ML ALGORITHM FOR TRAINING MODEL PARAMETERS OF AN ML MODEL UNTIL A STOPPING CRITERION IS REACHED

108 — OUTPUTTING THE MODEL PARAMETERS REPRESENTATIVE OF THE TRAINED ML MODEL

## FIG. 1B

110

| 111 | RECEIVING DATA REPRESENTATIVE OF A PLURALITY OF CANDIDATE MOLECULE DRUGS INCLUDING, FOR EACH CANDIDATE MOLECULE DRUG, A SET OF PHYSIOCHEMICAL VALUES AND A SET OF PK VALUES ASSOCIATED WITH A DOSAGE REGIMEN AND TARGET REPRESENTING SAID EACH CANDIDATE MOLECULE DRUG |

112 — FOR EACH OF THE CANDIDATE MOLECULE DRUGS

113 — APPLYING CORRESPONDING SET OF PHYSIOCHEMICAL VALUES AND SET OF PK VALUES ASSOCIATED WITH THE DOSAGE REGIMEN AND TARGET TO A ML MODEL

114 — RECEIVING, FROM THE ML MODEL, A CLASSIFICATION FOR THE CANDIDATE MOLECULE DRUG

117
SELECT NEXT CANDIDATE MOLECULE DRUG

115 — CLASSIFICATION INDICATE CANDIDATE MOLECULE DRUG MEETS DESIRED CONDITIONS RELATED TO PHARMACOLOGICAL/PHARMACODYNAMIC EFFECTS?

NO

YES

116 — ADD MOLECULE DRUG TO SHORTLIST OF CANDIDATE MOLECULE DRUGS

118 — OUTPUT SHORTLIST OF CANDIDATE MOLECULE DRUGS FOR USE IN DOWNSTREAM PROCESSING SUCH AS IN-VITRO WET LAB ANALYSIS, IN-VIVO TRIALS, HIGH THROUGHPUT SCREENING LABORATORY ANALYSIS, AND/OR FURTHER DRUG OPTIMISATION ANALYSIS

*FIG. 1C*

~120

121 — RECEIVING A PLURALITY OF MOLECULE DRUGS, EACH MOLECULE DRUG REPRESENTED BY A SET OF PHYSIOCHEMICAL VALUES

122 — RECEIVING ONE OR MORE SETS OF PHYSIOCHEMICAL VALUES CORRESPONDING TO THE TARGET

123 — RECEIVING A SET OF DOSAGE REGIMENS

124 — GENERATING, FOR EACH DESIRED DOSAGE REGIMEN, ONE OR MORE UNIQUE PAIRS OF MOLECULE DRUG AND TARGET DATA

FOR EACH DOSAGE REGIMEN AND EACH PAIR OF MOLECULE DRUG / TARGET DATA

125 — APPLYING THE SET OF PHYSIOCHEMICAL VALUES FOR THE MOLECULE DRUG AND TARGET OF THE MOLECULE DRUG AND TARGET PAIR TO A PBPK MODEL

126 — COMPUTING USING THE PBPK MODEL A SET OF PK VALUES AND THE CORRESPONDING PHARMACOLOGICAL/PHARMACODYNAMIC VALUE(S) FOR THE APPLIED MOLECULE DRUG AND TARGET DATA PAIR

127 — ADDING EITHER:

A) A TRAINING DATA INSTANCE TO A TRAINING DATASET BASED ON THE SET OF PHYSIOCHEMICAL PROPERTIES FOR THE MOLECULE DRUG AND THE COMPUTED SET OF PK VALUES WITH ANNOTATIONS ACCORDING TO THE ONE OR MORE DESIRED CONDITIONS IN RELATION TO THE COMPUTED PHARMACOLOGICAL/PHARMACODYNAMIC VALUES; OR

B) A CANDIDATE MOLECULE DRUG INFERENCE DATA INSTANCE TO A CANDIDATE MOLECULE DRUG INFERENCE DATASET BASED ON THE SET OF PHYSIOCHEMICAL PROPERTIES FOR THE MOLECULE DRUG AND THE COMPUTED SET OF PK VALUES

128 — OUTPUTTING OR STORING THE TRAINING DATASET OR CANDIDATE MOLECULE DRUG INFERENCE DATASET

## FIG. 1D

**FIG. 2A**

CLASSIFY EACH VIRTUAL CANDIDATE BASED
ON TO % > 90% OR < 90%

SCENARIOS OF INTEREST

- FIXED DOSE
- FIXED DOSING SCHEME
- FIXED CHARGE

203a

DECISION TREE-BASED ML

IDENTIFY OPTIMAL DRUG TARGET
PROPERTIES NEEDED FOR 90% TO

203b

203c

*FIG. 2A*
*(CONT.)*

**FIG. 2B**

FIG. 2C

EP 4 625 423 A1

FIG. 2C
(CONT.)

**FIG. 2D**

**FIG. 2D**
*(CONT.)*

EP 4 625 423 A1

**FIG. 2E**

FIG. 2F

GENERATION OF MOLECULAR PHYSIOCHEMICAL PROPERTIES
253

STEP 2

GENERATE LARGE NUMBER OF VIRTUAL COMPOUNDS WITH DISTINCT PHYSICOCHEMICAL PROPERTIES AND TARGET BINDING AFFINITIES.

mAb1           Ag1

| MW | Q | ... | KD | | $t_{1/2}$ | $T_0$ |
|----|---|-----|----|---|-----------|-------|

mAbn           Agn

| MW | Q | ... | KD | | $t_{1/2}$ | $T_0$ |
|----|---|-----|----|---|-----------|-------|

TO
FIG. 2G (CONT.)

**FIG. 2G**

FIG. 2G (CONT.)

*FIG. 3*

400

402 404

408 406

# FIG. 4

Europäisches
Patentamt

European
Patent Office

Office européen
des brevets

# EUROPEAN SEARCH REPORT

**Application Number**

EP 24 31 5210

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EITRICH T. ET AL: "Classification of Highly Unbalanced CYP450 Data of Drugs Using Cost Sensitive Machine Learning Techniques", JOURNAL OF CHEMICAL INFORMATION AND MODELING, vol. 47, no. 1, 8 December 2006 (2006-12-08), pages 92-103, XP093203527, US ISSN: 1549-9596, DOI: 10.1021/ci6002619 * Abstract * * page 93, column 2 * * table 2 * * page 96, column 1 * * page 93, column 1 * * figure 7 * | 1-29 | INV. G16C20/30 G16C20/70 |
| A | SELVARAJ CHANDRABOSE ET AL: "Artificial intelligence and machine learning approaches for drug design: challenges and opportunities for the pharmaceutical industries", MOLECULAR DIVERSITY, SPRINGER INTERNATIONAL PUBLISHING, CHAM, vol. 26, no. 3, 23 October 2021 (2021-10-23), pages 1893-1913, XP037878399, ISSN: 1381-1991, DOI: 10.1007/S11030-021-10326-Z [retrieved on 2021-10-23] * Abstract; page 1898, "Machine learning for high-throughput screening"; Figure 7 * | 1-29 | TECHNICAL FIELDS SEARCHED (IPC) G16C |

-----

-/--

The present search report has been drawn up for all claims

| Place of search | Date of completion of the search | Examiner |
|---|---|---|
| Munich | 17 September 2024 | Schmidt, Karsten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
............................................................................
& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 24 31 5210

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SEO SANGMIN ET AL: "Prediction of GPCR-Ligand Binding Using Machine Learning Algorithms", COMPUTATIONAL AND MATHEMATICAL METHODS IN MEDICINE, vol. 2018, 1 January 2018 (2018-01-01), pages 1-5, XP093204209, ISSN: 1748-670X, DOI: 10.1155/2018/6565241 Retrieved from the Internet: URL:http://downloads.hindawi.com/journals/cmmm/2018/6565241.xml> * the whole document * ----- | 1-29 | |
| | | | TECHNICAL FIELDS SEARCHED (IPC) |

The present search report has been drawn up for all claims

| Place of search | Date of completion of the search | Examiner |
|---|---|---|
| Munich | 17 September 2024 | Schmidt, Karsten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................................
& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **Z. LI** ; **D. K. SHAH**. Two-pore physiologically based pharmacokinetic model with de novo derived parameters for predicting plasma PK of different size protein therapeutics.. *Journal of pharmacokinetics and pharmacodynamics*, 2019, 305-218